# EUROPÄISCHE PATENTANMELDUNG

(11) **EP 4 425 004 A2**
(43) Veröffentlichungstag der Anmeldung: **04.09.2024**
(21) Anmeldenummer: 24190152.9
(22) Anmeldetag: 31.10.2014
(51) Int. Cl.: F16C 1/28

(54) **FLEXIBLER KATHETER MIT EINER ANTRIEBSWELLE**

(30) Priorität: 01.11.2013 EP 13191307
(62) Teilanmeldung aus: 20192267.1
(71) Anmelder: ECP Entwicklungsgesellschaft mbH, 52074 Aachen (DE)
(72) Erfinder: Scheckel, Mario, 52074 Aachen (DE); Schumacher, Jörg, 52074 Aachen (DE)
(74) Vertreter: Pfenning, Meinig & Partner mbB

(57) **Zusammenfassung**

Die Erfindung betrifft einen flexiblen Katheter (1) mit einer Antriebswelle (2), einer die Antriebswelle (2) umgebenden Hülse (6) und einem die Antriebswelle (2) und die Hülse (6) umgebender Mantel (7), wobei die Antriebswelle, die Hülse (6) und der Mantel (7) biegsam sind, wobei die Antriebswelle (2) an einem proximalen Ende der Antriebswelle (2) ein Kupplungselement (5) aufweist zum Verbinden der Antriebswelle (2) mit einem Antriebsmotor (18), wobei die Antriebswelle (2) zumindest bereichsweise aus einer Legierung besteht, die zu jeweils mindestens 10% Gewichtsanteil Chrom, Nickel und Cobalt beinhaltet. Die Erfindung betrifft außerdem eine Blutpumpenanordnung mit einem solchen Katheter.

## Beschreibung

Die Erfindung betrifft einen flexiblen Katheter mit einer biegsamen Antriebswelle gemäß dem Oberbegriff des Hauptanspruchs sowie eine Blutpumpenanordnung mit einem solchen Katheter.

Derartige Katheter werden typischerweise dazu verwendet, innerhalb eines Körpers eines Menschen oder eines Tieres ein Drehmoment oder eine Drehbewegung zu erzeugen bzw. zu übertragen. Die Antriebswelle verläuft axial entlang der Längsausdehnung des Katheters zwischen einem proximalen Ende des Katheters und einem distalen Ende des Katheters. Typischerweise ist ein proximales Ende der Antriebswelle außerhalb des Körpers mit einem Antriebsmotor verbunden, um das Drehmoment bzw. die Drehbewegung zu erzeugen und auf die Antriebswelle zu übertragen. Am distalen Ende der Antriebswelle ist typischerweise ein Drehelement bzw. Funktionselement drehfest mit der Antriebswelle verbunden, das für die jeweilige Anwendung geeignet ausgestaltet ist. Bei dem Funktionselement kann es sich beispielsweise um eine Fräse, einen Rotorablator oder um einen Pumpenrotor zur Förderung von Blut handeln.

Für viele Anwendungen ist es erforderlich, den Katheter entlang eines gewundenen Pfades durch den Körper zu führen, beispielsweise entlang bzw. innerhalb von Blutgefäßen, um das distale Ende des Katheters an einer gewünschten Stelle innerhalb des Körpers, beispielsweise innerhalb einer Herzkammer, für die Dauer der jeweiligen Anwendung zu positionieren. Neben der hierfür erforderlichen Biegsamkeit und Flexibilität des Katheters müssen in der Regel noch weitere Kriterien erfüllt sein. Beispielsweise ist es in manchen Anwendungen erforderlich, dass mit der Antriebswelle Drehbewegungen mit einer sehr hohen Drehzahl erzeugt bzw. übertragen werden, beispielsweise kann die erforderliche Drehzahl mehr als 10.000, mehr als 20.000 oder sogar mehr als 30.000 Umdrehungen pro Minute betragen, wie beispielsweise im bereits genannten Fall der Blutförderung. In Fällen, in denen die Drehbewegung über einen längeren Zeitraum, also für mehrere Stunden, Tage oder sogar Wochen erzeugt werden muss, wie dies ebenfalls bei der Blutförderung der Fall sein kann, werden außerdem besonders hohe Anforderungen an die mechanische und an die chemische Belastbarkeit des Katheters gestellt, insbesondere an die Antriebswelle. Materialermüdung und Schädigungsprozesse an der Antriebswelle und an anderen Komponenten des Katheters sollten möglichst langsam und außerdem möglichst auf vorhersehbare und kontrollierbare Weise voranschreiten. In kritischen Anwendungen, wie beispielsweise der Blutförderung, müssen Risse und Brüche der Antriebswelle im Betrieb mit möglichst großer Sicherheit ausgeschlossen werden können. Hierbei ist es ungünstig, wenn die flexible Antriebswelle in einer zu harten, die Welle verschleißenden Umhüllung betrieben wird.

Andererseits muss im Falle eines dennoch auftretenden Versagens der Welle mit möglichst großer Sicherheit ausgeschlossen werden, dass sich die dabei typischerweise aufspleißenden Enden der Welle mit hoher Drehzahl durch den üblicherweise aus Kunststoff bestehenden Mantel des Katheters arbeiten. In einem solchen Fall würden sich die Enden der Welle frei im Blutgefäß drehen.

Es ist somit die Aufgabe der vorliegenden Erfindung, einen flexiblen Katheter mit einer biegsamen Antriebswelle vorzuschlagen, der möglichst sicher ist und sich möglichst auch für einen Dauerbetrieb bei hohen Drehzahlen eignet. Außerdem ist eine Blutpumpenanordnung vorzuschlagen, die ebenfalls möglichst sicher ist und sich möglichst auch für einen Dauerbetrieb bei hohen Drehzahlen eignet.

Diese Aufgabe wird gelöst durch ein Katheter gemäß dem Hauptanspruch sowie durch einen Katheter und eine Blutpumpenanordnung gemäß den nebengeordneten Ansprüchen. Bevorzugte Ausführungsformen und Weiterentwicklungen ergeben sich mit den abhängigen Ansprüchen.

Wie im Folgenden detailliert beschrieben wird, offenbart diese Patentanmeldung mehrere Aspekte, wobei jeder dieser Aspekte ein Teil einer zusammenhängenden Erfindung ist. Andererseits stellt jeder der Aspekte auch für sich genommen bereits eine eigenständige Erfindung dar. Die Aspekte können also unabhängig voneinander realisiert werden (und stellen jeweils für sich genommen besondere Weiterentwicklungen eines gattungsgemäßen Katheters gemäß dem Oberbegriff des Hauptanspruchs dar) und können außerdem beliebig miteinander kombiniert werden, um einen gattungsgemäßen Katheter bzw. eine Blutpumpenanordnung mit einem gattungsgemäßen Katheter synergetisch zu verbessern. So kann beispielsweise ein gattungsgemäßer Katheter gemäß einem dieser Aspekte ausgestaltet sein und kann gleichzeitig auch gemäß einem (oder mehreren) weiteren Aspekt(en) ausgestaltet sein. Dieser Katheter ist dann eine besonders vorteilhafte Ausführungsform eines Katheters gemäß dem erstgenannten Aspekt. Jeder der Aspekte ermöglicht somit auch eine Weiterentwicklung jedes anderen Aspektes.

Der erste dieser Aspekte betrifft das Material bzw. die Materialeigenschaften der Antriebswelle, der zweite Aspekt die geometrische Ausgestaltung der Antriebswelle, der dritte Aspekt die Ausgestaltung der Hülse, der vierte Aspekt die Verbindung zwischen der Antriebswelle und dem Antriebsmotor, der fünfte Aspekt die Lagerung der Antriebswelle und der sechste Aspekt ein Schmiermedium für die Antriebswelle. Jeder dieser Aspekte trägt zur Verbesserung der Belastbarkeit und der Sicherheit des Katheters bzw. der Blutpumpenanordnung bei.

Ein gattungsgemäßer flexibler Katheter umfasst demnach eine Antriebswelle, eine die Antriebswelle umgebende Hülse und einen die Antriebswelle und die Hülse umgebenden Mantel, wobei die Antriebswelle, die Hülse und der Mantel biegsam sind. An einem proximalen Ende der Antriebswelle weist die Antriebswelle ein Kupplungselement bzw. einen Kupplungskopf auf zum Verbinden der Antriebswelle mit einem Antriebsmotor.

Typischerweise beträgt die (axiale) Gesamtlänge des Katheters zwischen 50 cm und 200 cm, in der Regel liegt die Gesamtlänge in einem Bereich zwischen 80 cm und 150 cm. Typischerweise liegen die (axialen) Gesamtlängen der Antriebswelle, der Hülse und des Mantels ebenfalls jeweils innerhalb eines dieser Bereiche. Die Flexibilität bzw. die Biegsamkeit des Katheters, also insbesondere der Antriebswelle, der Hülse und des Mantels, sollen ausreichend sein, um den Katheter mit einem Krümmungsradius in einem Bereich zwischen 20 mm und 40 mm, vorzugsweise in einem Bereich zwischen 25 mm und 35 mm, insbesondere von etwa 30 mm elastisch krümmen zu können. Bei einer solchen Krümmung sollen sich insbesondere die bei Betriebsdrehzahl drehende Antriebswelle und die Hülse möglichst nur elastisch verformen, es soll also zu möglichst keinen bleibenden (plastischen) Verformungen oder Veränderungen der Antriebswelle oder der Hülse kommen. Insbesondere soll eine solche elastische Krümmung mit dem genannten Krümmungsradius auch bei einer etwa U-förmigen Krümmung des Katheters von etwa 180° möglich sein, bei der der Katheter also beispielsweise entlang eines axialen Abschnitts des Katheters mit einer Länge von etwa (je nach Krümmungsradius) 80 mm bis 150 mm, typischerweise von etwa 100 mm bis 120 mm, durchgängig gekrümmt ist. Derartige Krümmungen des Katheters treten beispielsweise dann auf, wenn der Katheter durch den Aortenbogen in die linke Herzkammer verläuft. Typischerweise kommt es darüber hinaus durch die rhythmische Herzaktion zu einer rhythmischen Änderung des beschriebenen Krümmungsradius, wobei sich weiterhin die Position der Krümmung gegenüber dem Katheter rhythmisch ändern kann.

In vielen Fällen ist es nicht erforderlich, dass der Katheter entlang seiner gesamten axialen Längsausdehnung eine derartige Flexibilität aufweist. Es kann bereits ausreichend sein, dass dies in einem bestimmten axialen Abschnitt (oder mehreren axialen Abschnitten) gegeben ist. Oftmals, wie im Fall der Blutförderung, beispielsweise wenn ein distales Ende des Katheters in einer Herzkammer platziert werden muss, weist zumindest ein distales Endstück oder ein distales Teilstück des Katheters eine derartige Flexibilität auf. Dieses distale Endstück oder Teilstück kann beispielsweise eine axiale Länge in einem der oben genannten Längenbereiche aufweisen.

Wie weiter unten näher beschrieben wird, darf in manchen Fällen die Biegsamkeit und Flexibilität des Katheters bzw. der Antriebswelle auch nicht zu groß werden, insbesondere in solchen axialen Abschnitten der Antriebswelle, die distal oder proximal außerhalb der Hülse verlaufen oder aus der Hülse austreten, so dass in zumindest einem dieser Abschnitte zu einem bestimmten Grad eine bereichsweise Versteifung der Antriebswelle vorteilhaft oder, je nach Anforderungen der jeweiligen Anwendung, sogar erforderlich sein kann. Durch eine derartige Versteifung kann vorteilhafterweise eine Vibrationsminimierung erzielt werden, wodurch auch das Risiko einer Hämolyse gesenkt werden kann.

Gemäß dem ersten Aspekt der Erfindung kann die Antriebswelle des Katheters vollständig oder zumindest bereichsweise aus einer Legierung bestehen, die zu jeweils mindestens 10% Gewichtsanteil Chrom, Nickel und Cobalt beinhaltet. Vorzugsweise beinhaltet die Legierung zu mindestens 30% Gewichtsanteil Nickel, vorzugsweise aber nicht mehr als 40% Gewichtsanteil Nickel. Vorzugsweise beinhaltet die Legierung zu mindestens 30% Gewichtsanteil Cobalt, vorzugsweise aber nicht mehr als 40% Gewichtsanteil Cobalt. Vorzugsweise beinhaltet die Legierung zu 15% Gewichtsanteil Chrom, vorzugsweise aber nicht mehr als 25% Gewichtsanteil Chrom. Die Legierung beinhaltet vorzugsweise außerdem Molybdän, vorzugsweise mindestens 5% Gewichtsanteil, vorzugsweise aber nicht mehr als 15% Gewichtsanteil Molybdän.

Beispielsweise kann die Legierung als Legierungsanteile etwa 35% Gewichtsanteil Nickel, etwa 35% Gewichtsanteil Cobalt, etwa 20% Gewichtsanteil Chrom und etwa 10% Gewichtanteil Molybdän enthalten. Diese Legierungsanteile der Legierung können jeweils auch um bis zu 3% Gewichtsanteile größer oder geringer sein oder jeweils um bis zu 2% größer oder geringer sein. Die Legierungsanteile dieser Elemente können den Legierungsanteilen dieser Elemente in der Legierung MP35N^{®} oder den Legierungsanteilen dieser Elemente in der Legierung 35NLT^{®} entsprechen oder hiervon jeweils um bis zu 2% Gewichtsanteile nach oben oder nach unten abweichen oder hiervon jeweils um bis zu 1% nach oben oder nach unten abweichen. Die Legierung kann außerdem weitere Legierungselemente beinhalten. Diese können entsprechend denen der Legierung MP35N^{®} oder denen der Legierung 35NLT^{®} ausgewählt und bemessen sein.

Vorzugsweise handelt es sich bei der Legierung um MP35N^{®} oder 35NLT^{®} oder ist auf entsprechende (oder gleiche) Weise, d.h. mit entsprechenden (oder gleichen) Verfahrensschritten und mit entsprechenden (oder gleichen) Verfahrensparametern, hergestellt worden wie MP35N^{®} bzw. wie 35NLT^{®}. Beispielsweise kann vorgesehen sein, dass die Legierung der Antriebswelle bzw. die Antriebswelle als Ganzes kaltverfestigt ist oder durch Anwendung einer (Hoch-)Kaltverformung oder Kaltverfestigung hergestellt bzw. geformt worden ist. Beispielsweise beträgt ein Kaltverfestigungsgrad des Werkstoffs der Antriebswelle und/oder der Hülse zwischen 35% und 70% und/oder zwischen 40% und 60%. Hieraus kann sich eine Zugfestigkeit des Werkstoff in einem Bereich zwischen 1900 MPa und 2200 MPa ergeben.

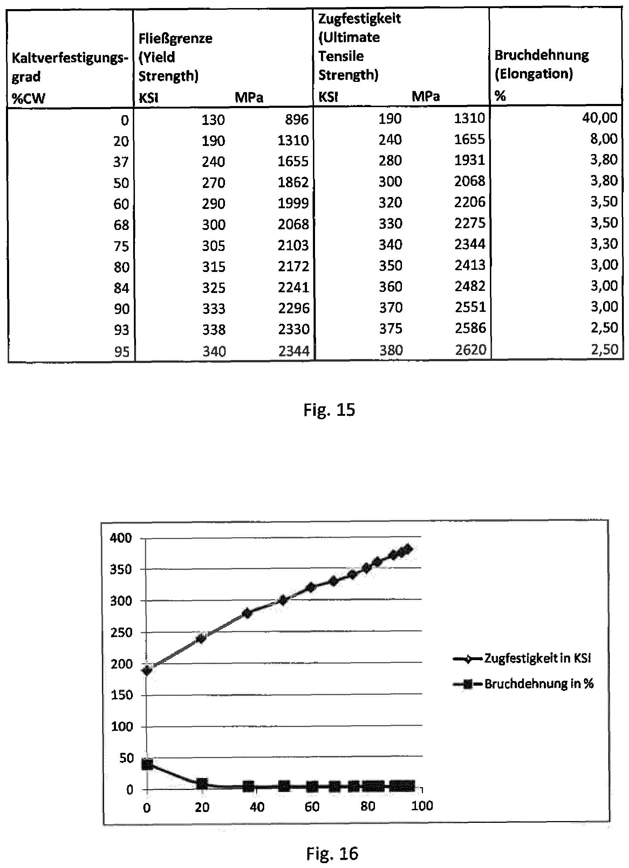

In Figuren 16 und 17 sind die Zusammenhänge zwischen Fließgrenze, Zugfestigkeit, Bruchdehnung und Kaltverfestigungsgrad am Beispiels des Werkstoffs 35NLT exemplarisch dargestellt (basierend auf Angaben des Herstellers Fort Wayne Metals). Es zeigt sich an diesem Beispiel, dass verschiedene Wärmebehandlungszustände und Kaltverfestigungsgrade eines Werkstoffs generell zu sehr unterschiedlichen Werkstoffeigenschaften führen können. Diese stellen sich oft erst im Nachhinein als ungeeignet für eine flexible Antriebswelle heraus.

Eine hohe Kaltverfestigung ist beispielsweise nicht unkritisch, da dies zu einer Herabsetzung der maximalen Bruchdehnung wie auch der Zähigkeit des Materials führen kann. Hierdurch kann die Biegewechselfestigkeit der Antriebswelle wie auch der erreichbare Biegeradius der Welle im Betrieb negativ beeinfluss werden. Eine geringe Kaltverfestigung geht andererseits mit einer relative geringen Härte und Zugfestigkeit des Werkstoffs einher. Eine zu geringe Härte hat unmittelbaren Einfluss auf das Verschleißverhalten der Welle und damit auch auf ihre Dauerfestigkeit, und kann beispielsweise einen erhöhten Abrieb und Verschleiß im Betrieb zur Folge habe. Dies ist insbesondere im Fall einer Gleitpaarung, wie sie für flexible Wellen typisch ist, kritisch. Eine geringere Zugfestigkeit hat eine geringe Biegewechselfestigkeit zur Folge.

Da also unterschiedliche Optimierungsziele der Antriebswelle auf divergierende Werkstoffkennwerte treffen, ist die Optimierung einer stabilen und langlebigen flexiblen Antriebswelle hochkomplex, so dass sich hierfür kein standardmäßiges und sinnvoll verwendbares Optimierungsverfahren oder offensichtliches Parameterfenster ergibt.

Überraschenderweise hat sich aber gezeigt, dass Antriebswellen, die ganz oder zumindest bereichsweise aus einem Material bestehen, das eine Zugfestigkeit in einem Bereich zwischen 1800 N/mm² und 2400 N/mm², vorzugsweise von 2034 bis 2241 N/mm² (also von 295 KSI bis 325 KSI), aufweist, zu guten Ergebnissen führen. Bei dem Material kann es sich insbesondere um eine der hier beschriebenen Legierungen handeln, welche also zu jeweils mindestens 10% Gewichtsanteil Chrom, Nickel und Cobalt beinhaltet. Außer diesen Legierungen kommen aber auch andere Werkstoffe für die Antriebswelle in Frage, wie beispielsweise metallische und nicht-metallische Werkstoffe, insbesondere auch Kunststoffe und Verbundwerkstoffe.

Eine Antriebswelle die vollständig oder zumindest bereichsweise aus einer solchen Legierung bzw. einem solchen Material besteht, eignet sich auch für Anwendungen bei sehr hohen Drehzahlen und langem Dauerbetrieb, so dass es mit einer solchen Antriebswelle auch möglich ist, die eingangsgenannten Drehzahlbereiche für eine längere Zeitdauer aufrechtzuerhalten. Typischerweise sind die Drehmomente, die solch hohen Drehzahlen mittels der Antriebswelle übertragen, jedoch relativ gering, insbesondere bei der Blutförderung, wobei das Drehmoment zum Antrieb eines expandierten Pumpenrotors auf Grund des größeren Durchmessers typischerweise höher ist. Auch wenn die Verwendung der Legierungen MP35N^{®} bzw. wie 35NLT^{®} aufgrund ihrer Belastbarkeit und ihres Korrosionswiderstandes beispielsweise für verschiedene medizinische Instrumente, wie beispielsweise ein Stylet, bekannt sein mag, überrascht ihre Eignung für biegsame Antriebswellen aufgrund der beschriebenen speziellen Anforderungen, insbesondere bei hoher Drehzahl, langer Betriebsdauer und starker Krümmung, insbesondere vor dem Hintergrund, dass für die Anwendung als Blutpumpe durchaus mehr als 500 000 000 vollständige Lastwechsel in Extremfällen mehr als 1000 000 000 Lastwechsel auftreten können.

Bislang wurden für die Antriebswelle, insbesondere für Blutpumpen, Legierungen mit einem relativ hohen Eisenanteil oder Titananteil verwendet, um eine hohe Belastbarkeit zu erzielen. Wie sich jedoch im Rahmen der vorliegenden Erfindung herausgestellt hat, kann bzw. sollte sogar möglichst auf einen hohen Anteil von Eisen und Titan verzichtet werden, um einen Dauerbetrieb bei hohen Drehzahlen zu ermöglichen. Der Gewichtsanteil an Eisen und Titan ist vorzugsweise sogar relativ gering gewählt, vorzugsweise jeweils weniger als 2% Gewichtsanteil, oder sogar jeweils weniger als 1% Gewichtsanteil. Im Prinzip kann auf Eisen und Titan als Legierungsanteile völlig verzichtet werden, entsprechend einem Gewichtsanteil von jeweils weniger als 0,1%.

Gemäß dem ersten Aspekt kann die Antriebswelle vollständig oder zumindest bereichsweise aus einer Legierung bestehen, die einen Gewichtsanteil von Eisen aufweist, der weniger als 2% beträgt oder vorzugsweise weniger als 1% beträgt oder besonders bevorzugt weniger als 0,1% beträgt. Gemäß dem ersten Aspekt kann die Antriebswelle vollständig oder zumindest bereichsweise aus einer Legierung bestehen, die einen Gewichtsanteil von Titan aufweist, der weniger als 2% beträgt oder vorzugsweise weniger als 1% beträgt oder besonders bevorzugt weniger als 0,1% beträgt.

Vorzugsweise bestehen die Antriebswelle, die Hülse, der Mantel und/oder ggf. vorhandene Lagerelemente möglichst aus biokompatiblen Materialien oder besteht zumindest eine Außenflächen der jeweiligen Komponente aus einem biokompatiblen Material.

Entsprechend dem zweiten Aspekt der Erfindung kann die Antriebswelle einen sich innerhalb der Antriebswelle axial erstreckenden Hohlraum aufweisen. Bei der Antriebswelle kann es sich dann also um eine Hohlwelle handeln. Der Hohlraum kann sich innerhalb der Antriebswelle entlang einer gesamten Längsausdehnung der Antriebswelle erstrecken. Durch den Hohlraum kann eine hohe Biegsamkeit der Antriebswelle bei gleichzeitiger relativ großer Torsionssteifheit erzielt werden. Die Biegsamkeit kann weiter gesteigert werden, wenn die Antriebswelle eine Mehrzahl bzw. eine Vielzahl koaxialer Windungen aufweist, die den Hohlraum der Antriebswelle wendelförmig umlaufen. Außerdem können durch die Windungen Torsions- und Biegespannungen in axiale Zug- bzw. in Druckspannungen umgewandelt werden, wodurch sich die Belastung der Antriebswelle reduzieren kann. Es ist außerdem möglich, dass die Windungen der Antriebswelle in zwei oder mehr koaxialen Lagen der Antriebswelle angeordnet sind. Dann haben die Windungen innerhalb verschiedener koaxialer Lagen vorzugsweise gegenläufige Wicklungsrichtungen. Auf diese Weise können sich durch Torsionsspannungen hervorgerufene Zug- und Druckspannungen zwischen den Lagen vollständig oder zumindest teilweise gegenseitig ausgleichen bzw. kompensieren. Insgesamt können so außerdem auch Biegespannungen in der Antriebswelle verringert werden.

Bei den Windungen der Antriebswelle handelt es sich typischerweise um Windungen eines gewundenen Drahtes oder mehrerer entsprechend gewundener Drähte, innerhalb jeder Lage kann die Antriebswelle genau einen oder mehrere solche Drähte umfassen, beispielsweise 1 bis 8 Drähte, vorzugsweise 4 bis 6 Drähte, besonders bevorzugte 5 Drähte. Der Draht bzw. die Drähte bestehen vorzugsweise aus der oben beschriebenen Legierung. Der Draht bzw. die Drähte haben typischerweise jeweils Durchmesser in einem Bereich von etwa 0,09 mm bis etwa 0,21 mm, vorzugweise von etwa 0,135 mm bis etwa 0,165 mm. Ein Außendurchmesser der Antriebswelle liegt typischerweise in einem Bereich von etwa 0,53 mm bis etwa 1,32 mm, vorzugsweise in einem Bereich von etwa 0,79 mm bis etwa 0,97 mm. Insbesondere sind Außendurchmesser der Antriebswellen unter 1 mm bevorzugt. Ein Innendurchmesser der Antriebswelle liegt typischerweise in einem Bereich von etwa 0,17 mm bis etwa 0,39 mm, vorzugsweise in einem Bereich von etwa 0,25 mm bis etwa 0,31 mm. Im Fall von zwei konzentrischen Lagen berühren sich axial benachbarte Windungen der inneren Lage gegenseitig, wohingegen axial benachbarte Windungen der äußeren Lage vorzugsweise sich gegenseitig nicht berühren (jeweils bei krümmungsfreier Ausrichtung der Antriebswelle), sondern einen axialen Abstand in einem Bereich von etwa 0,018 mm bis etwa 0,042 mm, vorzugsweise von etwa 0,027 mm bis etwa 0,033 mm, aufweisen.

Durch einen kleinen Außendurchmesser der Antriebswelle ist auch ein kleiner Außendurchmesser des Katheters realisierbar, wodurch eine geringere Traumatisierung des Gewebes an der Punktionsstelle erzielt werden kann. Weitere Vorteile, die sich durch einen geringen Außendurchmesser der Antriebswelle erzielen lassen sind geringere Reibungs- und Abriebprobleme aufgrund einer geringeren Umfangsgeschwindigkeit der Antriebswelle, geringere Vibrationsprobleme aufgrund einer geringerer Masse der Antriebswelle sowie infolgedessen geringere Störungen von Motorstromsignalen durch Vibrationen und beispielsweise eine geringere Gefahr, dass sich eventuell vorhandene Kalzifizierungen innerhalb der Blutgefäße von der Gefäßwand lösen und mit möglicherweise lebensbedrohlichen Folgen für den Patienten in den Kreislauf gelangen.

Überaschenderweise hat sich somit herausgestellt, dass die Übertragung ausreichend hoher Drehmomente, beispielsweise zum Antreiben eines expandierbaren Pumpenrotors im expandierten Zustand, auch mit den hier genannten geringen Außendurchmessern der Antriebswelle von weniger als 1 mm über längere Zeit möglich sind. Hierbei haben sich insbesondere auch die oben angegebenen speziellen Bereiche für die Durchmesser der Drähte im Falle einer aus derartigen Drähten aufgebauten Welle als besonders vorteilhaft erwiesen, wobei sich außerdem herausgestellt, dass der optimale Bereich für die Durchmesser der Einzeldrähte nicht-trivial mit dem Außendurchmesser der Antriebswelle zusammenhängt.

Ferner kann vorgesehen sein, dass die Windungen der Antriebswelle durch Anwendung einer (Hoch-)Kaltverformung oder Kaltverfestigung hergestellt bzw. geformt worden sind, um die Elastizität und Haltbarkeit der Antriebswelle zu verbessern.

Es ist möglich, dass der Hohlraum vollständig oder innerhalb axialer Abschnitte der Antriebswelle mit einem Verstärkungsmaterial ausgefüllt ist, um die Steifigkeit und Stabilität der Antriebswelle in dem jeweiligen axialen Abschnitt einzustellen und (ggf. bereichsweise) zu vergrößern. Wie bereits im Zusammenhang mit dem ersten Aspekt der Erfindung erläutert, ist neben einer ausreichenden Biegsamkeit der Antriebswelle gleichzeitig auch eine ausreichende Steifigkeit der Antriebswelle für einen sicheren Betrieb des Katheters, insbesondere bei hohen Drehzahlen und langer Betriebsdauer, erforderlich, beispielsweise um eine stabile Rotation der Antriebswelle zu ermögliche, insbesondere in axialen Abschnitten der Antriebswelle, die distal oder proximal außerhalb der Hülse verlaufen (distales bzw. proximales Endstück der Antriebswelle). Auf diese Weise ergänzen sich der erste und der zweite Aspekt der Erfindung auf synergetische Weise. In einer bevorzugten Ausführungsform ist demnach vorgesehen, dass ein distales Endstück der Antriebswelle und/oder ein proximales Ende bzw. Endstück der Antriebswelle versteift ist bzw. sind. Vorzugsweise weist das versteifte distale bzw. proximale Ende bzw. Endstück eine Länge zwischen 10 mm bis 60 mm auf, besonders bevorzugt eine Länge zwischen 20 mm und 50 mm. Vorzugsweise ist die Antriebswelle in solchen Bereichen versteift, in denen (zusätzlich zur Hülse oder statt bzw. an Stelle der Hülse) Lagerelemente zur axialen und/oder radialen Lagerung der Antriebswelle angeordnet sind. Vorteilhaft kann es außerdem sein, die Antriebswelle in dem Bereich zu versteifen, in denen die Antriebswelle proximal in die Hülse ein- bzw. austritt und folglich nicht in der Hülse geführt ist. Vorteilhaft kann es außerdem sein, die Antriebswelle in dem Bereich zu versteifen, in denen die Antriebswelle distal in die Hülse ein- bzw. austritt. Gerade in diesen Übergangsbereichen können durch eine Versteifung der Antriebswelle Biege- und andere Belastungen, wie beispielsweise Schwingungsbelastungen, der Antriebswelle reduziert werden.

Als Verstärkungsmaterialen zur Versteifung der Antriebswelle eignen sich Materialien, die sich durch eine hohe Steifigkeit einerseits und gleichzeitig durch eine verhältnismäßig hohe elastische Verformbarkeit auszeichnen. Insbesondere soll das Verstärkungsmaterial bzw. das Versteifungsmaterial sämtliche Biegungen, die das Katheter bzw. der Pumpenkopf des Katheters während der Implantation und während des Betriebs ausgesetzt ist, tolerieren. Beispielsweise kommt ein nicht rostender, austenitischer Stahl als Verstärkungsmaterial in Frage, beispielsweise ein Stahl gemäß der Werkstoffnummer DIN 1.4310.

Alternativ oder zusätzlich zu dem beschriebenen Verstärkungsmaterial kann eine entsprechende Versteifung durch (axiales und/oder radiales) Verschwei-ßen oder Verlöten (axial bzw. radial) benachbarter der Windungen der (wendelförmigen) Antriebswelle erreicht werden. Außerdem ist es möglich, dass eine gewisse (und unter Umständen ausreichende) Versteifung der Antriebswelle durch das distale Funktionsmodul, das typischerweise drehfest auf einem Außenumfang der Antriebsachse befestigt ist, wie beispielsweise ein Pumpenrotor, erreicht werden kann.

Gemäß dem dritten Aspekt der Erfindung kann die Hülse als eine Lagerspirale mit einer Mehrzahl von Windungen ausgestaltet sein. Die Windungen der Lagerspirale umlaufen die Antriebswelle in axialer Richtung in der Art einer Wendel. Beispielsweise kann die Lagerspirale ein aufgewickeltes Flachband sein. Das Flachband hat vorzugsweise um einen Faktor von mindestens 3, vorzugsweise um einen Faktor von 6, größere Breite (axial gemessen) als Dicke (radial gemessen). Typischerweise liegt die Breite der Windungen in einem Bereich von etwa 0,36 mm bis etwa 0,84 mm, vorzugsweise in einem Bereich von etwa 0,54 mm bis etwa 0,66 mm. Die Dicke der Windungen liegt typischerweise in einem Bereich von etwa 0,06 mm bis etwa 0,14 mm, vorzugsweise in einem Bereich von etwa 0,09 mm bis etwa 0,11 mm. Ferner liegt ein Innendurchmesser der Hülse typischerweise in einem Bereich zwischen etwa 0,6 mm und etwa 1,4 mm, vorzugsweise in einem Bereich von etwa 0,9 mm bis etwa 1,1 mm. Ein Außendurchmesser der Hülse liegt typischerweise in einem Bereich von etwa 0,72 mm bis etwa 1,68 mm, vorzugsweise in einem Bereich von etwa 1,08 mm bis etwa 1,32 mm. Eine Steigung der Lagerspirale liegt vorzugsweise in einem Bereich von etwa 0,43 bis etwa 0,98, vorzugsweise in einem Bereich von etwa 0,63 bis 0,77, wobei der Innendurchmesser der Hülse mit dem Außendurchmesser der flexiblen Antriebswelle korrespondiert, insbesondere also größer ist als der Außendurchmesser der Antriebswelle.

Vorzugsweise weist die Lagerspirale, falls sie als gewickeltes Flachband ausgestaltet ist, möglichst geringe Fertigungstoleranzen bezüglich einer (axialen) Verkippung der Windungen relativ zur Längsachse der Lagerspirale auf (im geraden Zustand ohne Krümmung der Lagerspirale). Vorzugsweise beträgt die Verkippung weniger als 10°, besonders bevorzugt weniger als 5°. Vorzugsweise bildet die Innenfläche der Hülse bzw. der Windungen der Lagerspiralen somit zylinderförmige Teilflächen aus anstelle von konischen Teilflächen (Verkippung). Eine Verkippung der Windungen zur Längsachse führt zu einer Verkleinerung der zur Verfügung stehenden Lagerfläche und zu einer größeren Druckbelastung der Antriebswelle. Vorzugsweise sind die seitlichen Kanten des Flachbandes möglichst abgerundet, um Druckspitzen auf die Antriebswelle möglichst zu vermeiden. Vorzugsweise beträgt der Krümmungsradius der Kanten 0,04 mm oder mehr.

Die Hülse kann vollständig oder zumindest bereichsweise aus einer Legierung bestehen. Die Beschreibung der Legierung der Antriebswelle kann entsprechend auch auf die Legierung der Hülse übertragen werden. Insbesondere kann die Hülse ganz oder zumindest bereichsweise aus demselben Werkstoff, beispielsweise der gleichen Legierung, bestehen wie die Antriebswelle.

Bei der Verwendung gleiches gleichen Werkstoffs für die Antriebswelle und die Hülse konnten im Labortest unter unterschiedlicher pulsatiler Last und mit Biegeradien deutlich unter 50mm sehr gute Ergebnisse im Dauertest erreicht werden. Dies ist in vielerlei Hinsicht überraschend. Beispielsweise wird nämlich aus Gründen der Patientensicherheit empfohlen, die flexible Welle in einem im Vergleich zur Antriebswelle verhältnismäßig harten, verschleißfesten Werkstoff zu gestalten, um zu verhindern, dass sich die Antriebswelle, beispielsweise bei einem Wellenbruch, der üblicherweise zu einem Aufspleißen der Welle im Bruchbereich führt, im Folgebetrieb durch die Hülse und den noch weicheren Mantel des Katheters reibt und dann offen im Blutgefäß dreht. Außerdem wird im klassischen Maschinenbau üblicherweise von der Verwendung gleicher Werkstoffe als Gleitpartner bzw. Reibpartner abgeraten, da es in diesem Fall zum sog. "Fressen" der Werkstücke kommen kann, welches daher rührt, dass sich einzelne Moleküle der beiden Gleitpartner miteinander verbinden und dann aus dem Molekülverband des einen Teils herausgerissen werden können. Als besonders kritisch wird dabei angesehen, dass die Vorhersage, welches der beiden Teile verschleißt, schwierig oder sogar unmöglich sei. Die hier vorgeschlagene Verwendung gleicher Werkstoffe für eine schnelldrehende flexible Welle und eine darum befindliche Lagerspirale ist deshalb für den Fachmann überraschend.

Der vierte Aspekt der Erfindung betrifft die Ausgestaltung des proximalen Kupplungselements bzw. Kupplungskopfes der Antriebswelle, die überaschenderweise ebenfalls die Sicherheit des Katheters und dessen Eignung zur Daueranwendung wesentlich verbessern kann, insbesondere wenn dieser Aspekt mit einem der anderen Aspekte kombiniert wird. Die Grundidee des vierten Aspektes besteht darin, dass sich axiale Druck- und Zugspannungen in der Antriebswelle oftmals wesentlich reduzieren lassen, wenn die Verbindung zwischen dem Kupplungselement der Antriebswelle, das selbst möglichst starr ist und dreh-, zug-, und druckfest mit der Antriebswelle verbunden ist, und einem hierzu korrespondieren Kupplungselement des Antriebsmotors zwar drehfest ist, in axialer Richtung aber Ausgleichsbewegungen zwischen dem Kupplungselement der Antriebswelle und dem Kupplungselement des Antriebsmotor möglich sind. Hierzu können die Kupplungselemente von Antriebswelle und Antriebsmotor miteinander korrespondierende axiale Gleitflächen aufweisen, welche typischerweise parallel zur (lokalen) Drehachse bzw. der Längsachse des jeweiligen Kupplungselements verlaufen. Die Form dieser axialen Gleitflächen bzw. deren Außen- oder Innenkontur ändert sich also in axialer Richtung (also entlang der Drehachse bzw. Längsachse) nicht. Beispielsweise kann das Kupplungselement der Antriebsachse die Form eines Vierkants oder eines anderen Profilstücks aufweisen, welches eine in axialer Richtung, also entlang seiner Längsausdehnung bzw. der Drehachse, gleichbleibende Querschnittsfläche (definiert senkrecht zur Drehachse bzw. Längsachse) bzw. Außenkontur aufweist. Das Kupplungselement des Antriebsmotors kann entsprechend als eine korrespondierend ausgestaltete Aufnahme für den Vierkant bzw. das Profilstück ausgestaltet sein.

Wie bereits erwähnt, kann der Katheter an einem distalen Ende der Antriebswelle einen fest mit der Antriebswelle verbundenden Pumpenrotor aufweisen beispielsweise zur Blutförderung. Der Pumpenrotor kann, je nach Konfiguration, Ausgestaltung und Anstellwinkel einer Beschaufelung des Pumpenrotors, beispielsweise zur Förderung des Blutes nach proximal (proximale Förderrichtung, d.h. in Richtung des proximalen Endes des Katheters) oder nach distal (distale Förderrichtung, d.h. in Richtung des distalen Endes des Katheters) eingerichtet sein. Der fünfte Aspekt der Erfindung betrifft eine axiale Lagerung des Pumpenrotors, bei der ein Axiallager des Katheters auf die Förderrichtung Pumpenrotors so abgestimmt ist, dass axiale Lagerkräfte vor allem bzw. ausschließlich als axiale Zugkräfte (und weniger oder nicht als axiale Druckkräfte) auf die Antriebswelle wirken. Überaschenderweise kann hierdurch die Belastung der Antriebswelle, besonders bei hohen Drehzahlen, deutlich verringert werden. Darüber hinaus hat sich überraschenderweise gezeigt, dass bei einer derartigen Gestaltung der Blutpumpe die Blutschädigung durch den Pumpbetrieb geringer ist. Hierbei ist vorgesehen, dass im Fall einer proximalen Förderrichtung das Axiallager proximal zum Pumpenrotor angeordnet und ausgestaltet ist, einer nach distal gerichteten Axialverschiebung (ausgelöst durch die proximale Förderwirkung des Pumpenrotors) der Antriebswelle entgegenzuwirken. Im Fall distaler Förderrichtung ist das Axiallager distal zum Pumpenrotor angeordnet und ausgestaltet, einer nach proximal gerichteten Axialverschiebung der Antriebswelle entgegenzuwirken.

Beispielsweise kann das Axiallager ein erstes Axiallagerelement und ein zweites Axiallagerelement aufweisen, wobei das erste Axiallagerelement drehfest mit der Antriebswelle verbunden ist und das zweite Axiallagerelement fest mit der Hülse oder mit dem Mantel verbunden ist. Das erste Axiallagerelement und das zweite Axiallagerelement weisen einander zugewandte, vorzugsweise ringförmige, Gleitflächen (welche auch als Stoßflächen oder als Stirnflächen bezeichnet werden können) auf, die eine Axialverschiebung der Antriebswelle bei einem gegenseitigen Berühren in zumindest einer Richtung blockieren. Die genannten Gleitflächen überlappen somit einander in radialer Richtung. Das erste Axiallagerelement kann als eine radiale Verbreiterung der Antriebswelle ausgebildet sein, aber auch als ein Ring, der auf der Antriebswelle, beispielsweise durch Krimpen, befestigt ist. Bei dem zweiten Axiallagerelement kann es sich gleichzeitig um ein Radiallagerelement handeln, beispielsweise mit einer der Antriebswelle zugewandten, vorzugsweise zylindrisch ausgestalteten und koaxial zur Drehachse der Antriebswelle angeordneten Gleitfläche.

Vorzugsweise weist zumindest eine der genannten Gleit- bzw. Stoßflächen, typischerweise zumindest die Gleitfläche des ersten Lagerelements des Axiallagers, eine Profilierung auf, so dass die beiden Gleitflächen im Zusammenspiel mit einem (fluiden) Schmiermedium ein hydrodynamisches Gleitlagers ausbilden. Als Schmiermedium kommt bevorzugt das weiter unten beschriebene Schmiermedium in Frage. Die Profilierung hat die Funktion, Bugwellen bzw. Druckwellen des Schmiermediums zwischen den beiden Gleitflächen zu erzeugen, wobei diese Wellen die Antriebswelle im Drehbetrieb umlaufen. Überraschenderweise konnte durch diese Gestaltung der Gleitflächen der entstehende Abrieb in diesem Bereich um mehr als 50% reduziert werden.

Beispielsweise kann die Profilierung der jeweiligen Gleitfläche mehrere, vorzugsweise 6 bis 24, Erhebungen und/oder Vertiefungen umfassen, welche vorzugsweise jeweils eine Höhe bzw. eine Tiefe von etwa 0,03 mm bis etwa 0,1 mm aufweisen können. Typischerweise sind die Erhöhungen und/oder Vertiefungen gleichmäßig entlang einer Umlaufrichtung bzw. Umfangrichtung der jeweiligen Gleitfläche über diese Gleitfläche verteilt angeordnet. Die Erhebungen können einander gleichen, ebenso können die Vertiefungen einander gleichen. Die Erhebungen können seitlich an die Vertiefungen angrenzen und umgekehrt. Insbesondere kann die Profilierung als eine (entlang der Umfangrichtung) alternierende Abfolge der Erhebungen und Vertiefungen ausgestaltet sein. Beispielsweise können die Erhebungen und/oder die Vertiefungen als Rippen bzw. als Rillen ausgestaltet sein, die typischerweise ausgehend von einem der Antriebswelle zugewandten inneren Rand der Gleitfläche sich in Richtung eines der Antriebswelle abgewandten äußeren Randes der Gleitfläche erstrecken. Typischerweise verlaufen die Rillen bzw. Rippen genau von dem inneren Rand bis genau zum äußeren Rand und haben dann also eine Länge, die der radial gemessenen Breite der jeweiligen Gleitfläche entspricht.

Die Rippen bzw. die Rillen weisen typischerweise eine Breite (in Umfangrichtung gemessen) in einem Bereich von etwa 0,08 mm bis etwa 0,5 mm auf. In radialer Richtung kann die Breite der Rippen bzw. Rillen konstant sein oder sich verändern. Typischerweise weist die Profilierung entlang der Umlaufrichtung der Gleitfläche abwechselnd Vertiefungen bzw. Rillen und eine Erhebung bzw. Rippen auf. Haben dann die Rillen eine konstante Breite, dann verbreitern sich die Rippen typischerweise radial nach außen. Derartige Ausführungsformen lassen sich häufig besonders einfach durch Fräsen herstellen. Haben andererseits die Rippen eine konstante Breite, dann verbreitern sich die Rillen typischerweise radial nach außen. Es ist aber auch möglich, dass sich sowohl die Rippen wie auch Rillen radial nach außen verbreitern. Die letzte Ausführungsform läßt sich häufig besonders einfach mittels Laserschneiden herstellen. Die Rillen bzw. Rippen können auch in Bereichen spiralig ausgebildet sein, sich also auf einer gekrümmten Bahn (beispielsweise eine Kreisbahn) vom inneren Rand bis zum äußeren Rand der Gleitfläche erstrecken.

Der Katheter kann die oben genannten Lagerelemente sowie weitere Lagerelemente zur radialen und/oder axialen Lagerung der Antriebswelle umfassen. Als Materialien für die Lagerelemente kommen jeweils beispielsweise Zirkonoxid (ZrO₂, auch als Zirconiumdioxid, Zirkoniumoxid, Zirkonoxid bezeichnet), insbesondere mit Yttrium stabilisiertes Zirkonoxid, Aluminiumoxid (AlOₓ, typischerweise Al₂O₃), Keramiken sowie die im Zusammenhang mit dem ersten Aspekt beschriebene Legierung in Frage.

Gemäß dem sechsten Aspekt der Erfindung ist ein Zwischenraum bzw. Zwischenspalt zwischen der Antriebswelle und der Hülse mit einem Schmiermedium, das biokompatible und vorzugsweise auch physiologisch ist, ausgefüllt. Bei diesem Schmiermedium kann es sich beispielsweise um destilliertes Wasser oder um eine wässrige Lösung handeln, beispielsweise eine Kochsalzlösung und/oder eine Glucoselösung. Die Lösung kann eine Konzentration an Kochsalz aufweisen, die physiologisch ist bzw. 0,9% beträgt. Es kann aber auch eine isotonische Kochsalzlösung oder sogenannte Ringerlösung vorgesehen werden. Dadurch, dass das Schmiermedium biokompatibel ist, kann einerseits der Aufbau des Katheters vereinfacht werden, da ein Austritt des Schmiermediums in den Körpers nicht unbedingt vermieden werden muss. Sofern für die Antriebswelle, die Hülse und die Lagerelemente die hier vorgeschlagenen Materialien verwendet werden, sind diese Komponenten chemisch relativ stabil gegenüber einer Korrosion durch diese (relativ korrosiven) Schmiermedien, so dass der Einsatz dieser Schmiermedien die Sicherheit und die Eignung des Katheters zum Dauerbetrieb praktisch nicht beeinträchtigt. Die Verwendung von Kochsalzlösung ist insofern besonders vorteilhaft, da derartige Lösung vom Patienten in der Regel sehr gut und ohne Nebenwirkungen vertragen wird, insbesondere auch bei Vorliegen einer Diabeteserkrankung des Patienten.

Die hier vorgeschlagene Blutpumpenanordnung umfasst einen Katheter hier vorgeschlagener Art sowie einen Antriebsmotor zur Erzeugung der Drehbewegung bzw. des Drehmoments. Zwischen dem Antriebsmotor, bzw. dem bereits oben genannten Kupplungselement des Antriebsmotors und dem Kupplungselement bzw. -kopf der Antriebswelle besteht eine drehfeste und vorzugsweise axial verschiebbare Verbindung. Bezüglich Letzterem wird auf die diesbezügliche Beschreibung im Zusammenhang mit dem vierten Aspekt verwiesen. Der Antriebsmotor kann dazu eingerichtet sein, hohe Drehzahlen zu erzeugen, beispielsweise Drehzahlen in einem Bereich zwischen 10.000 und 40.000 Umdrehungen pro Minute. Das mit dem distalen Endstück der Antriebswelle drehfest verbunden Funktionselement ist als Pumpenrotor ausgestaltet. Der Katheter weist an seinem distalen Ende ein Pumpengehäuse auf, in dem der Pumpenrotor angeordnet ist. Das Pumpengehäuse kann beispielsweise derart ausgebildet sein, dass das Pumpengehäuse (beispielsweise unter Beaufschlagung mit einer zum proximalen (bzw. distalen) Ende des Katheters hin wirkenden (Zug-)Kraft) von einem expandierten (bzw. komprimierten) Zustand in einen komprimierten (bzw. expandierten) Zustand überführt werden kann. Für die Einzelheiten wird auf die Druckschrift EP2399639 A1 verwiesen. Bei einer Verwendung der Pumpenanordnung kann beispielsweise vorgesehen sein, dass der Katheter mit seinem distalen Ende voran durch die Femoralarterie über den Aortenbogen in den linken Ventrikel des Herzens eingeschoben wird und das Pumpengehäuse im linken Ventrikel verbleibt. Ein mit dem Pumpengehäuse proximal verbundener Abströmschlauch, der dann durch die Aortenklappen typischerweise hindurch verläuft, kann beispielsweise das vom Pumpenrotor angetriebenes und aus dem Pumpengehäuse strömendes Blut in die Aorta leiten. Das proximale Ende des Katheters und insbesondere der Antriebswelle sowie der Antriebsmotor sind außerhalb des Körpers angeordnet.

Bei dieser und ähnlichen Anwendungen wirken verschiedene äußere Krafteinwirkungen und Biegewechselbelastungen auf die Antriebswelle und ggf. auf Lagerelemente des Katheters bzw. der Blutpumpenanordnung ein. Äußere Krafteinwirkungen und Biegewechselbelastungen können auf den Katheter übertragen werden beispielsweise durch eine Innenwand des Herzens, an der das Katheter ggf. anliegt bzw. abgestützt wird (bspw. über eine sogenannte Pigtail-Spitze), durch pulsatile Druckänderungen bzw. Strömungsänderungen des Bluts innerhalb einer Herzkammer bzw. eines Blutgefäßes, wie beispielsweise dem linken oder rechten Ventrikel oder der Aorta, durch eine Lage- bzw. Haltungsänderung des Körpers, insbesondere durch eine Rumpfbewegung oder eine (Bein-)Bewegung in der Nähe einer Punktionsstelle. Trotz dieser Belastungen kann mit dem vorgeschlagenen Katheter und der vorgeschlagene Blutpumpenanordnung Blut über längere Zeiträume, wie beispielsweise über Stunden, Tage oder gar Wochen auch bei hohen Drehzahlen des Pumpenrotors, beispielsweise im oben genannten Drehzahlbereich, gefördert werden, wie beispielsweise in der oben beschriebenen Verwendung der Blutpumpenanordnung.

Wie beispielsweise aus "The Sternotomy Hemopump. A second generation intraarterial ventricular assist device", Wampler RK et al., ASAIO J. 1993 Jul-Sep;39(3):M218-23, hervorgeht, können Wellenbrüche im Labor in der Regel nur unter pulsatilen Drucklasten und Biegeradien kleiner als 2inch (weniger als 50,8 mm) realistisch nachgestellt werden. Hierdurch wird insbesondere die Bedeutung multipler Belastung der Welle belegt. Es sind der Anmelderin außer der hier vorgeschlagenen Pumpenanordnung keine Pumpen mit flexibler Welle bekannt, die unter pulsatiler Last im Aortenbogen erfolgreich über längere Zeit eingesetzt worden sind. Dies auf die bis dato erfolglose Bearbeitung des Problems der flexiblen Welle zurückzuführen. Bislang wurde außerdem, insbesondere in der oben genannten Veröffentlichung von Wampler et al., die Verwendung einer 3-lagigen Welle anstelle einer 2-lagigen Welle als wesentlich für die Verbesserung der Lebensdauer der flexiblen Welle angesehen. Die hier vorgeschlagenen Antriebwellen weisen dahingegen auch in 2-lagiger Ausführung, und somit auch in Ausführungen mit erheblich geringerem Durchmesser als herkömmliche Antriebswellen, eine vergleichbar lange oder sogar noch erheblich längere Haltbarkeit und Belastbarkeit bei kleinen Biegeradien (kleiner als 50 mm) und pulsatiler Belastung auf als herkömmliche Antriebswellen.

Eine äußere Oberfläche der Antriebswelle kann überraschender Weise eine relativ große Rauheit RZ aufweisen. Beispielsweise kann die Rauheit RZ in einem Bereich von 0,01 µm bis 1µm, vorzugsweise in einem Bereich von 0,1 µm bis 0,8 µm, liegen. Beispielsweise kann die Rauheit RZ etwa 0,6 µm betragen. Die Tatsache, dass bei relativ großer Rauheit der Oberfläche der Antriebswelle sehr gute Ergebnisse im Dauertest erzielt werden konnten, ist überraschend, da aufgrund theoretischer Überlegungen eigentlich eine möglichst glatte Oberfläche zu bevorzugen wäre, um Verschleiß durch Reibung zu minimieren, insbesondere wenn als Schmiermedium, wie hier vorgeschlagen, eine relative korrosive Substanz, wie beispielsweise physiologische Kochsalzlösung oder Glucoselösung, verwendet wird, die in ihrer Schmierwirkung nicht annähernd an industrieübliche Schmierstoffe heranreicht, so dass die im klassischen Maschinenbau üblicherweise verwendeten Auslegungsprinzipien auch in diesem Punkt offenbar nicht direkt übertragbar sind.

Wie bereits beschrieben, umfasst ein flexibler Katheter hier vorgeschlagener Art eine Antriebswelle, eine die Antriebswelle umgebenden Hülse und einen die Antriebswelle und die Hülse umgebenden Mantel, wobei die Antriebswelle, die Hülse und der Mantel biegsam sind, wobei die Antriebswelle an einem proximalen Ende der Antriebswelle ein Kupplungselement aufweist zum Verbinden der Antriebswelle mit einem Antriebsmotor.

Außerdem kann die Antriebswelle einen Außendurchmesser von weniger als 1 mm aufweisen. Vorzugsweise besteht die Antriebswelle und/oder die Hülse zumindest bereichsweise aus einem Material, das eine Zugfestigkeit zwischen 1800 N/mm² und 2400 N/mm², vorzugsweise zwischen 2034 N/mm² und 2241 N/mm², aufweist. Die Antriebswelle und/oder die Hülse können zumindest bereichsweise aus einem nichtmetallischen oder einem metallischen Werkstoff bestehen. Im Fall eines metallischen Werkstoffs handelt es sich hierbei vorzugsweise um eine Legierung, wie weiter oben bereits beschrieben, die also zu jeweils mindestens 10% Gewichtsanteil Chrom, Nickel und Cobalt beinhaltet. Diese Legierung kann die weiter oben bereits beschriebenen Merkmale aufweisen. Die Antriebswelle und die Hülse können vollständig oder zumindest bereichsweise aus demselben Werkstoff bestehen. Ferner kann, wie weiter oben schon beschrieben worden ist, eine Oberfläche der Antriebswelle eine Rauheit zwischen 0,01 µm und 1 µm, vorzugsweise zwischen 0,1 µm und 0,8 µm, aufweisen. Selbstverständlich kann der Katheter alle der zuvor und im Folgenden beschrieben Merkmale und Merkmalskombinationen aufweisen.

Die genannten Aspekte der vorliegenden Erfindung werden im Folgenden anhand eines in Figuren 1 bis 16 schematisch dargestellten speziellen Ausführungsbeispiels eines Katheters hier vorgeschlagener Art und einer Blutpumpenanordnung hier vorgeschlagener Art näher erläutert. Es zeigt:
- Fig. 1: einen Katheter hier vorgeschlagener Art in einer seitlichen Ansicht,
- Fig. 2: eine Blutpumpenanordnung mit dem in Figur 1 gezeigten Katheter in einem implantierten Zustand,
- Fig. 3: axiale Abschnitte von Teilen einer Antriebswelle des Katheters aus Figur 1 in einer seitlichen Ansicht,
- Fig. 4: einen Querschnitt durch die in Figur 3 gezeigte Antriebswelle an der dort mit AA gekennzeichneten Stelle,
- Fig. 5: ein mit einem Verstärkungsmaterial versteiftes distales End- stück der Antriebswelle in einer seitlichen Ansicht,
- Fig. 6: einen Längsschnitt durch das in Figur 5 gezeigte Endstück an der dort mit AA gekennzeichneten Stelle,
- Fig. 7: eine Hülse des in Figur 1 gezeigten Katheters in einer Seitenansicht,
- Fig. 8: einen Querschnitt durch einen in Figur 7 mit A gekennzeichneten Teilbereich der dort gezeigten Hülse,
- Fig. 9: einen Längsschnitt durch den in Figur 1 gezeigten Katheter in dem dort mit Y gekennzeichneten axialen Teilabschnitt,
- Fig. 10: das in Figuren 5 und 6 dargestellte distale Endstück mit einem an diesem drehfest befestigten Pumpenrotor,
- Fig. 11: einen Längsschnitt durch den in Figur 1 gezeigten Katheter in dem dort mit Z gekennzeichneten axialen Teilabschnitt,
- Fig. 12: einen Längsschnitt durch ein Kupplungsmodul des in Figur 1 gezeigten Katheters, und
- Fig. 13: ein Ausführungsbeispiel eines Lagerelements eines in Figur 9 gezeigten Axiallagers in einer perspektivischen Darstellung,
- Fig. 14: ein weiteres Ausführungsbeispiel des in Figur 13 gezeigten Lagerelements ebenfalls in einer perspektivischen Darstellung,
- Fig. 15: Messwerte von Fließgrenze, Zugfestigkeit und Bruchdehnung für verschiedene Werte des Kaltverfestigungsgrads für den Werkstoff 35NLT, und
- Fig. 16: Diagrammatische Darstellung der in Figur 15 angegebenen Messwerte der Zugfestigkeit und der Bruchdehnung als Funktionen des Kaltverfestigungsgrad für den Werkstoff 35NLT.

In den Figuren sind widerkehrende bzw. einander entsprechende Merkmale mit den gleichen Bezugszeichen gekennzeichnet.

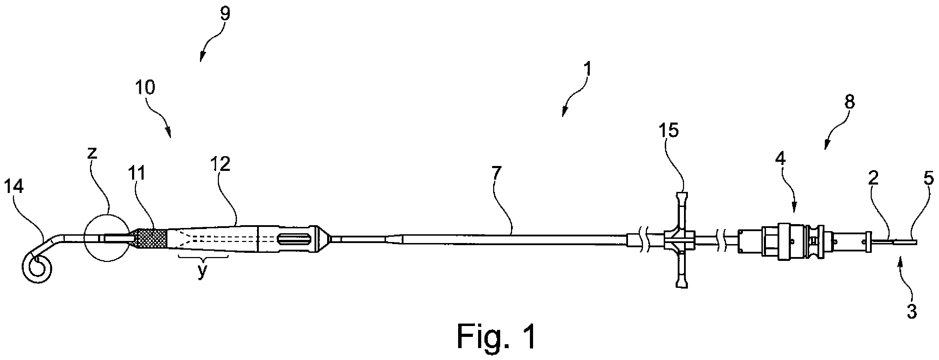

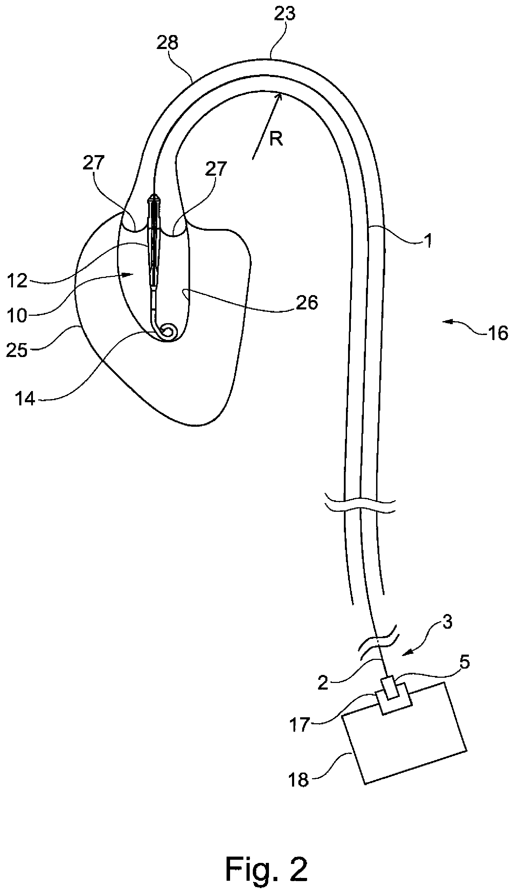

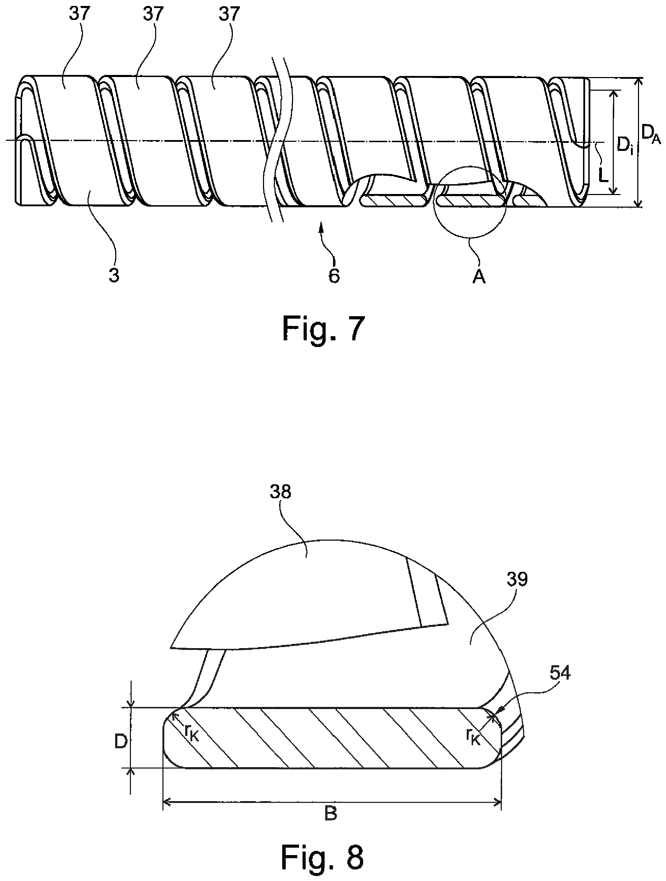

In Figur 1 ist eine spezielle Ausführungsform eines flexiblen Katheters 1 hier vorgeschlagener Art schematisch dargestellt. Der Katheter 1 umfasst eine biegsame Antriebswelle 2, von der in dieser Figur ein proximales Endstück 3 zu sehen ist, das aus einem proximalen Kupplungsmodul 4 (freitragend) herausragt und an dessen proximalen Ende die Antriebswelle 2 ein Kupplungselement 5 aufweist zum Verbinden der Antriebswelle 2 mit einem Antriebsmotor, vgl. Figur 2. Der Katheter 1 umfasst außerdem eine die Antriebswelle 2 umgebende und radial lagernde biegsame Hülse 6 (hier nicht gezeigt, siehe aber Figuren 7 bis 9, und einen die Antriebswelle 1 und einen die Hülse 6 umgebenden biegsamen Mantel 7. Während also das Kupplungsmodul 4 und das proximale Endstück 3 der Antriebswelle 2 an einem proximalen Ende 8 des Katheters 1 angeordnet sind, umfasst das Katheter 1 an einem distales Ende 9 des Katheters 1 einen Pumpenkopf 10 mit einem Pumpengehäuse 11, einem zum Pumpengehäuse 11 distal angeordneten Abschlussgehäuse 13 für die Antriebswelle 2 und einem proximal an das Pumpengehäuse 11 angrenzenden Abströmschlauch 12 (in Figur 1 sind innerhalb des Abströmschlauchs 12 verlaufende Elemente gestrichelt dargestellt). An dem Abschlussgehäuse 13 ist distal ein Stützelement 14 in der Form einer sogenannten Pigtail-Spitze angeordnet. Außerdem weist der Katheter 1 eine Schleuse 15 auf. Ihre Funktion ist es, den Pumpenkopf 10, wenn dieser in die Schleuse 15 hineingezogen wird, radial zu komprimieren. In diesem komprimierten Zustand kann der Pumpenkopf 10 beispielsweise anschließend durch eine Einführschleuse (in den Figuren nicht dargestellt) geführt und durch diese hindurch implantiert werden. Die Einführschleuse kann beispielsweise an einer Punktionsstelle am bzw. im Körper eines Patienten fixiert sein, um auf diese Weise den Katheter 1 an dieser Stelle ebenfalls abzustützen. In diesem Zusammenhang sei auch auf die Druckschrift EP2399639 A1 verwiesen.

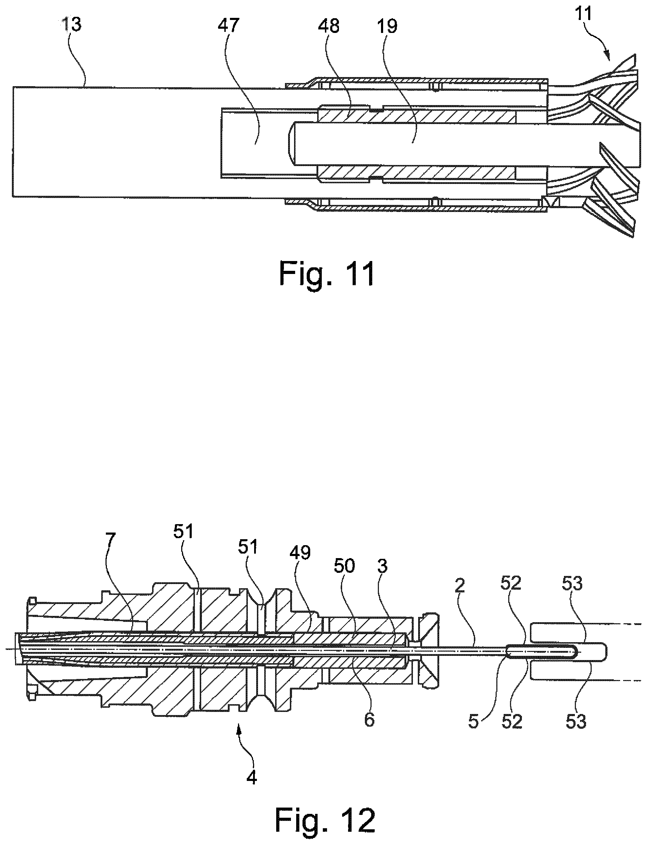

In Figur 2 ist dieses Katheter als Teil einer Blutpumpenanordnung 16 in einem implantierten Zustand stark schematisiert dargestellt. Gezeigt ist eine Verwendung bzw. Anwendung des Katheters 1 und der Blutpumpenanordnung 16, bei die Antriebswelle 2 des Katheter 1 über das Kupplungselement 5 drehfest (aber axial verschiebbar, siehe Beschreibung zu Figur 12) mit einem korrespondieren Kupplungselement 17 eines Antriebsmotor 18 der Blutpumpenanordnung 1 verbunden ist. Der Antriebsmotor 18 ist dazu eingerichtet, hohe Drehzahlen in einem Bereich zwischen 10.000 und 40.000 Umdrehungen pro Minute zu erzeugen.

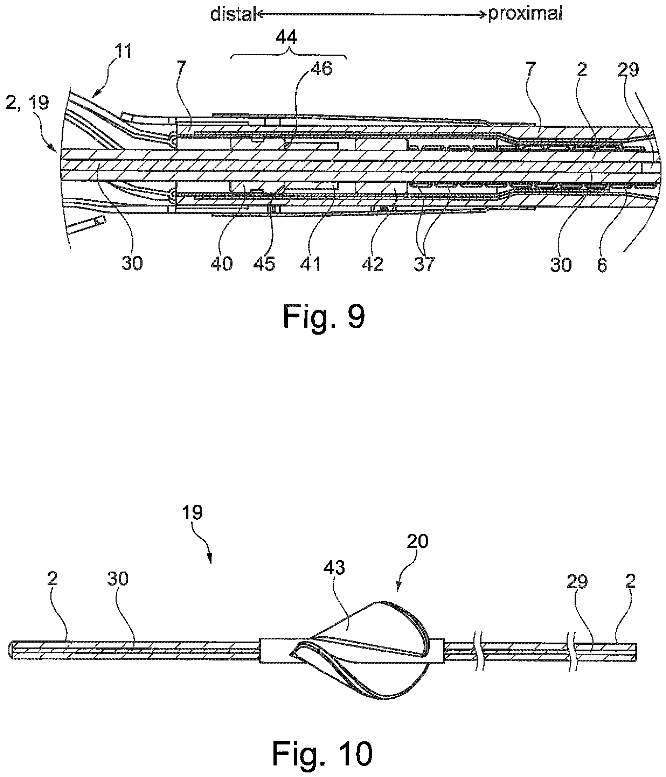

Wie in Figur 10 gezeigt ist, ist mit einem distalen Endstück 19 der Antriebswelle 2, ein als Pumpenrotor 20 ausgestaltetes Funktionselement drehfest verbunden. Der Pumpenrotor 20 ist innerhalb des Pumpengehäuses 11 angeordnet, welches in diesem Ausführungsbeispiel ausgebildet ist, dass es von einem radial expandierten Zustand in einen radial komprimierten Zustand überführt werden kann. Dies kann beispielsweise mit Hilfe der Schleuse 15 oder der oben genannten Einführschleuse erfolgen, vorzugsweise indem das Pumpengehäuse 11 unter Beaufschlagung mit einer zum proximalen Ende 8 des Katheters hin wirkenden (Zug-)Kraft zumindest teilweise in die jeweilige Schleuse gezogen wird und dabei entlang einer quer zur Längsrichtung verlaufenden Radialrichtung komprimiert wird. Durch eine umgekehrte Kraft kann das Pumpengehäuse 11 entsprechend aus dem komprimierten in den expandierten Zustand überführt werden. Auch an dieser Stelle sei auf die Druckschrift EP2399639 A1 verwiesen.

Bei der in Figur 2 gezeigten Verwendung der Pumpenanordnung 2 wird der Katheter 1 mit seinem distalen Ende 9 voran durch eine Punktionsstelle 21 in den Körper eines Patienten in dessen Femoralarterie 22 eingeführt und entlang dieser über den Aortenbogen 23 in das linke Ventrikel 24 des Herzens 25 eingeschoben. Das Pumpengehäuse 11 wird so in dem linken Ventrikel positioniert, dass es durch das Stützelement 14 an einer Innenwand 26 des linken Ventrikels 24 abgestützt wird und der Abströmschlauch 12 durch die Aortenklappen 27 hindurch in die Aorta 28 hinein verläuft. Das vom Pumpenrotor 20 angetriebene und aus dem Pumpengehäuse herausströmende Blut wird somit durch den Abströmschlauch 12 in die Aorta 28 geleitet. Das proximale Ende 8 des Katheters 1, das proximale Endstück 3 der Antriebswelle 2 sowie der Antriebsmotor 18 sind außerhalb des Körpers angeordnet.

Um diese Verwendung zu ermöglichen betragen in diesem Ausführungsbeispiel eine (axiale) Gesamtlänge des Katheters und eine (axiale) Gesamtlänge der Antriebswelle 2 jeweils etwa 150 cm (entsprechend einer implantierbaren Länge von etwa 140 cm), eine (axiale) Gesamtlänge des distalen Endes 9 des Katheters (einschließlich Pumpenkopf 12 und Stützelement 14) beträgt etwa 13,5 cm. Die Flexibilität bzw. die Biegsamkeit des Katheters 1, also insbesondere der Antriebswelle 2, der Hülse 6 und des Mantels 7 sind so groß, dass der Katheter 1, wie oben beschrieben worden ist, implantiert und betrieben werden kann. Hierzu müssen diese Komponenten zumindest innerhalb des distalen Endes 9 des Katheters mit dem typischen Krümmungsradius R des Aortenbogens 23 von etwa 30mm um 180° elastisch gekrümmt werden können, wie in Figur 2 gezeigt, ohne dass es hierbei zu plastischen Verformungen insbesondere der Antriebswelle 2 kommt.

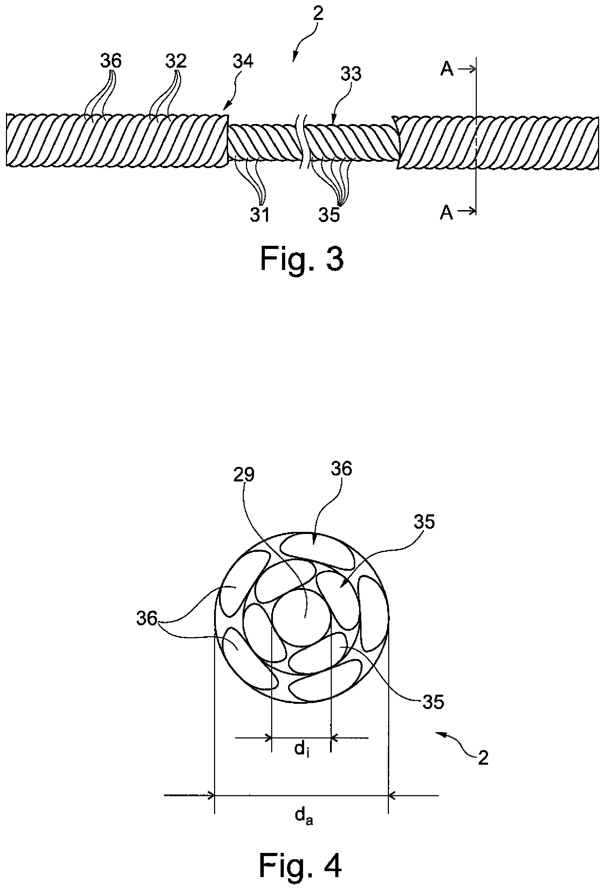

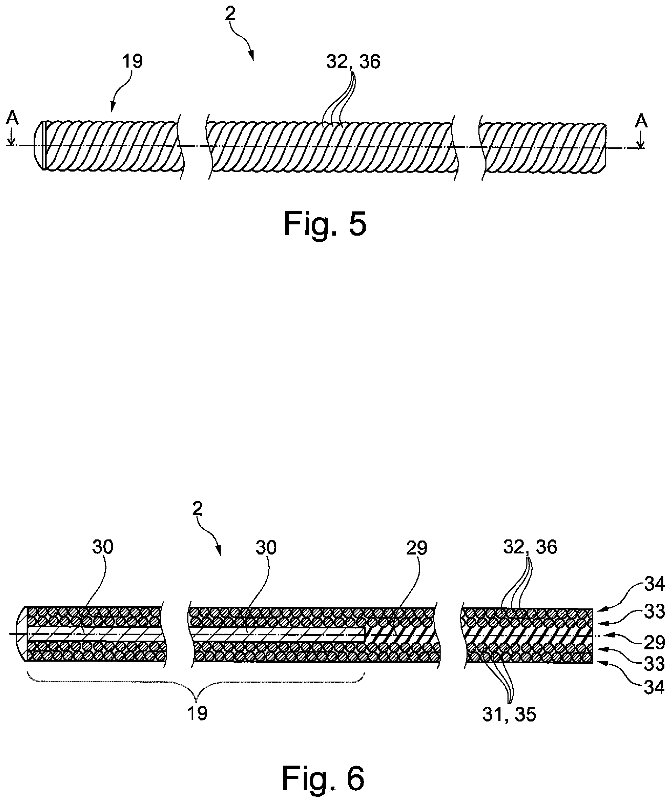

Um eine hohe Biegsamkeit der Antriebswelle 2 zu erreichen, ist diese, wie in Figuren 4 und 6 gezeigt ist, als eine Hohlwelle ausgestaltet und weist einen sich innerhalb der Antriebswelle 2 axial erstreckenden Hohlraum 29 auf. Der Hohlraum 29 erstreckt entlang der Gesamtlänge der Antriebswelle 2. Zumindest innerhalb des etwa 4,5 cm langen distalen Endstücks 19 der Antriebswelle ist dieser Hohlraum 29 aber vollständig mit einem Verstärkungsmaterial 30, einer sogenannten Seele, ausgefüllt, siehe Figuren 6, 9 und 10 und die zugehörige Beschreibung weiter unten, um hier eine ausreichende Steifigkeit und Schwingungsstabilität der Antriebswelle 2 bzw. des distalen Endstücks 19 der Antriebswelle zu erreichen.

Die Antriebswelle 2 weist eine Vielzahl koaxialer Windungen 31, 32 auf, die den Hohlraum 29 der Antriebswelle 2 wendelförmig umlaufen, um Torsions- und Biegespannungen in axiale Zug- bzw. in Druckspannungen umzuwandeln. Die Windungen 31, 32 sind in zwei koaxialen Lagen 33, 34 bzw. Schichten der Antriebswelle 2 angeordnet, wobei die Windungen 31 innerhalb der inneren Lage 33 koradial (mit gleichem Wickelradius) angeordnet sind und die Windungen 32 innerhalb der äußeren Lage koradial angeordnet sind. Die Windungen der 31 inneren Lage 33 haben in Bezug auf die Windungen der äußeren Lage 34 eine gegenläufige Wicklungsrichtung, so dass sich Zug- und Druckspannungen zwischen den Lagen kompensieren können. Im gezeigten Beispiel umfasst die Antriebswelle in der inneren Lage 33 vier koaxial und koradial um den Hohlraum 29 gewickelte Drähte 35 und in der äußeren Lage 34 fünf koaxial und koradial um den Hohlraum gewickelte Drähte, wobei sich axial benachbarte Windungen 31 der inneren Lage gegenseitig berühren, sich axial benachbarte Windungen (Wicklungspakete aus je fünf Drähten) 32 der äußeren Lage aber gegenseitig nicht berühren (jeweils bei krümmungsfreier Ausrichtung der Antriebswelle), sondern einen axialen Abstand von etwa 0,03 mm aufweisen. Ein Außendurchmesser dₐ der Antriebswelle beträgt im vorliegenden Beispiel etwa 0,88 mm und ein Innendurchmesser dᵢ etwa 0,28 mm. Die Drähte haben einen kreisrunden Querschnitt mit einem Durchmesser von etwa 0,15 mm. Im vorliegenden Beispiel ist die Umlaufrichtung der Windungen 36 der äußeren Lage 34 der bestimmungsgemäßen Drehrichtung der Antriebswelle 2 zur (proximalen) Blutförderung entgegengesetzt.

Vorliegend entspricht diese Drehrichtung dem Uhrzeigersinn (definiert für eine Blickrichtung vom proximalen zum distalen Ende der Antriebswelle). Das zu übertragende Drehmoment führt in diesem Fall dazu, dass die äußere Lage dazu tendiert, sich zusammen zu ziehen und zu verkürzen. Da die innere Lage 33 aufgrund ihres entgegengesetzten Wicklungsrichtung eine entgegengesetzte Tendenz aufweist, gleichen sich diese Tendenzen vorteilhafterweise weitgehend aus. Prinzipiell lässt sich diese gegenseitige Kompensation auch im umgekehrten Fall erreichen, wenn nämlich der Wicklungssinn der äußeren Lage zur Drehrichtung entspricht und der Wicklungssinn der inneren Lage der Drehrichtung der Antriebswelle entgegengesetzt ist.

Die Drähte 35, 36 der Antriebswelle 2 bestehen vollständig aus einer Legierung, welche als Legierungsanteile etwa 35% Gewichtsanteil Nickel, etwa 35% Gewichtsanteil Cobalt, etwa 20% Gewichtsanteil Chrom und etwa 10% Gewichtanteil Molybdän enthalten. Diese Legierungsanteile der Legierung können jeweils auch um bis zu 3% Gewichtsanteile größer oder geringer sein oder jeweils um bis zu 2% größer oder geringer sein. Insbesondere handelt es sich bei der Legierung in diesem Beispiel um 35NLT^{®}, es könnte sich aber genauso gut auch um MP35N^{®} handeln. Der Gewichtsanteil an Eisen in den Drähten ist somit weniger als 1% Gewichtsanteil und der Gewichtsanteil an Titan ist weniger als 0,1% Gewichtsprozent. Die Legierung und die Windungen 31, 32 der Antriebswelle sind unter Anwendung einer Hochkaltverformung und Kaltverfestigung hergestellt bzw. geformt worden. Als Verstärkungsmaterial 30 zur Versteifung der Antriebswelle 2 ist in diesem Beispiel ein nicht rostender, austenitischer Stahl gemäß der Werkstoffnummer DIN 1.4310 gewählt (X10CrNi18-8). Alternativ könnte als Verstärkungsmaterial auch jedes andere Material gewählt werden, dass die in diesem Zusammenhang oben angegebenen Anforderungen erfüllt.

In Figuren 7 und 8 ist die Hülse 6 dargestellt, die im gezeigten Beispiel als eine Lagerspirale mit einer Vielzahl von Windungen 37 ausgestaltet ist, wobei die Windungen 37 der Lagerspirale die Antriebswelle 2 in axialer Richtung in der Art einer Wendel umlaufen. Im vorliegenden Beispiel ist die Lagerspirale durch ein aufgewickeltes Flachband 38 gegeben. Das Flachband 38 hat eine um einen Faktor von etwa 6 größere Breite B (axial gemessen) als Dicke D (radial gemessen). Im vorliegenden Beispiel beträgt die Breite B der Windungen 37 0,6 mm und die Dicke D der Windungen 37 0,1 mm. Die Windungen 37 sind außerdem möglichst geringfügig relativ zur Längsachse L der Lagerspirale (im geraden Zustand ohne Krümmung der Lagerspirale) angewinkelt bzw. gekippt, möglichst um weniger als 5°, so dass eine durch die Windungen 37 gebildete Innenfläche 39 der Hülse 6 möglichst zylindrisch ist bzw. möglichst zylindrische Teilflächen ausbildet. Außerdem sind Vorzugsweise sind die seitlichen Kanten 54 des Flachbandes möglichst abgerundet mit einem Krümmungsradius rₖ von etwa 0,04 mm. Vorzugsweise beträgt der Krümmungsradius rₖ der Kanten 54 mehr als 0,04 mm. Ferner beträgt ein Innendurchmesser Dᵢ der Hülse 6 etwa 1 mm und ein Außendurchmesser D_{A} der Hülse 6 etwa 1,2 mm und eine Steigung von etwa 0,7. Die Hülse 6 bzw. das Flachband 38 besteht in diesem Beispiel aus der gleichen Legierung wie die Drähte 35, 36 der Antriebswelle 2, vorliegend also aus 35NLT^{®}, könnte aber auch einem anderen der hierfür genannten Materialien gefertigt sein.

Die Antriebswelle 2 und die Hülse 6 könnten auch aus anderen Werkstoffen, als den hier genannten Legierungen bestehen. Vorzugsweise ist die Antriebswelle 2 aus demselben Werkstoff wie die Hülse 2 gefertigt. Außerdem kann eine Oberfläche der Antriebswelle 2 eine Rauheit von etwa RZ 0,6 aufweisen, wodurch überraschenderweise eine besonders gute Verschleißfestigkeit erreicht wird. Durch diese relativ leicht umsetzbaren Maßnahmen können überraschend gute Verschleißeigenschaften und somit eine hohe Betriebssicherheit erzielt werden.

In Figur 9 ist ein Längsschnitt durch den in Figur 1 mit Y bezeichneten axialen Abschnitt des Katheters 1 schematisch dargestellt. In diesem Abschnitt umfasst der Katheter 1 proximal zum Pumpenrotor 20 angeordnete Lagerelemente 40, 41, 42 zum radialen und axialen Lagern der Antriebswelle 2.

Die Anordnung und Ausgestaltung dieser Lagerelemente 40, 41, 42 ist auf den in Figur 10 gezeigten Pumpenrotor 20 des Katheters 1 abgestimmt. Dieser Pumpenrotor 20 weist eine Beschaufelung 43 auf, deren Konfiguration, Ausgestaltung und Anstellwinkel zur Förderung des Blutes nach proximal (proximale Förderrichtung, d.h. in Richtung des proximalen Endes des Katheters) eingerichtet ist. Die Lagerelemente 40 und 41 bilden ein zum Pumpenrotor 20 proximal angeordnetes Axiallager 44 aus. (Das Lagerelement 41 ist ein erstes Axiallagerelement des Axiallagers 44 und das Lagerelement 40 ist ein zweites Axiallagerelement des Axiallagers 44.) Das Axiallager 44 ist aufgrund der Ausgestaltung und Anordnung dieser (Axial-)Lagerelemente 40, 41 eingerichtet, einer nach distal gerichteten Axialverschiebung (hervorgerufen durch die proximale Förderwirkung des Pumpenrotors 20) der Antriebswelle 2 entgegenzuwirken. Auf diese Weise wirken im Betrieb der Blutpumpenanordnung axiale Lagerkräfte hauptsächlich als Zugkräfte auf die Antriebswelle 2 ein.

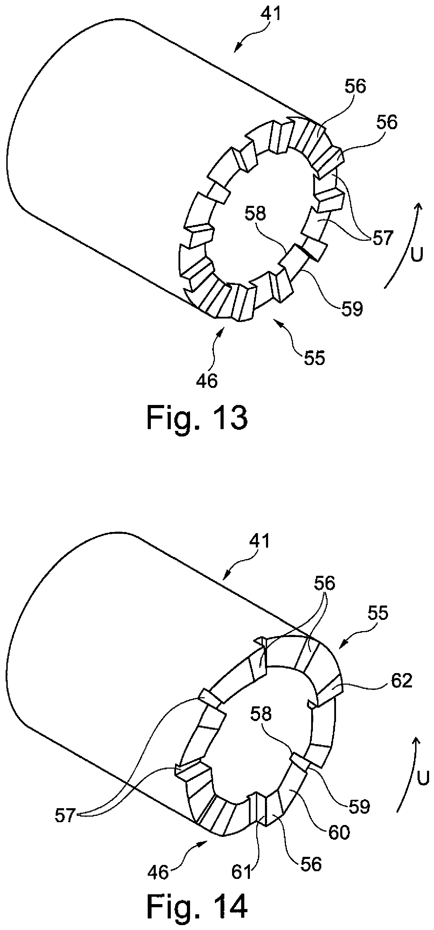

Das (erste) Lagerelement 41 ist vorzugsweise ringförmig ausgestaltet und drehfest, beispielsweise durch Krimpen, mit der Antriebswelle 22 verbunden. Das (zweite) Lagerelement 40 ist, ebenso wie das Lagerelement 42, dahingegen fest mit der Hülse 6 und mit dem Mantel 7 verbunden. Die Lagerelemente 40, 41 weisen einander zugewandte, ringförmige Gleitflächen 45 bzw. 46 auf, die eine Axialverschiebung der Antriebswelle 2 bei einem gegenseitigen Berühren in die distale Richtung blockieren. Die Gleitfläche 46 des (ersten) Lagerelementes 41 weist eine Profilierung auf, siehe Figuren 13 und 14 und die zugehörige Beschreibung unten, wodurch die Ausbildung eines stabilen Schmierfilms zwischen den beiden Gleitflächen 45, 46 begünstigt und eine Ausgestaltung des Axiallagers 44 als hydrodynamisches Gleitlager prinzipiell ermöglicht wird. Der Schmierfilm bzw. das hydrodynamische Lager wird in diesem Beispiel mit dem weiter unten beschriebenen Schmiermedium ausgebildet. Das Lagerelement 40 ist außerdem, wie auch das Lagerelement 42, als ein Radiallagerelement ausgestaltet mit jeweils einer der Antriebswelle 2 zugewandten, zylindrisch ausgestalteten und koaxial zur Drehachse der Antriebswelle 2 angeordneten Gleitfläche.

Wie in Figur 9 außerdem zu erkennen ist, ist die Antriebswelle 2 in den axialen Abschnitten, in denen sie distal aus der Hülse 6 austritt bzw. durch die Lagerelemente 40, 41, 42 gelagert ist, durch das Verstärkungsmaterial 30 versteift.

In Figur 11 ist ein Längsschnitt durch den in Figur 1 mit dem Bezugszeichen Z gekennzeichneten axialen Abschnitt des Katheters 1 schematisch dargestellt, der insbesondere das distal an das Pumpengehäuse 11 angrenzende Abschlussgehäuse 13 umfasst. Das Abschlussgehäuse 13 ist rohrförmig ausgestaltet und in einen distalen Lagerkanal 47 und ein darin angeordnetes Lagerelement 47 zur radialen Lagerung des distalen Endstücks 19 der Antriebswelle 2 auf. Der Hohlraum 47 ist insbesondere ausreichend groß bemessen, um axiale Ausgleichsbewegungen der Antriebswelle 2 zuzulassen.

In Figur 12 ist ein Längsschnitt durch das in Figur 1 gezeigte proximale Kupplungsmodul 4 schematisch dargestellt, das einen proximalen Lagerkanal 49 für das proximale Endstück 3 der Antriebswelle 2 aufweist, wobei das proximale Endstück 3 der Antriebswelle 2 den Lagerkanal 49 axial durchläuft und aus dem proximalen Kupplungsmodul 4 axial herausragt. In dem Lagerkanal 49 ist ein Lagerelement 50 zur radialen Stabilisierung bzw. Lagerung des proximalen Endstücks 3 der Antriebswelle 2 angeordnet. Die Hülse 6 erstreckt sich axial durch dieses Lagerelements 50 hindurch bis zu dessen proximalen Ende. Das Lagerelement 50 hat in dieser Ausführungsform die Funktion, die Hülse 6 von außen radial zu stabilisieren und abzustützen. In einer alternativen Ausführungsformen durchläuft die Hülse 60 das Lagerelement 50 nicht, sondern endet (von distal kommend) am distalen Ende des Lagerelements 50. In diesem Fall ist das Lagerelement 50 beispielweise als Gleitlager oder als Wälzlager ausgestaltet. Ebenso wie das distale Endstück 19 der Antriebswelle kann auch das proximale Endstück 3 durch das Verstärkungsmaterial 30 versteift sein, insbesondere in den axialen Abschnitten, in denen die Antriebswelle aus dem Lagerkanal 49 austritt bzw. durch das Lagerelement 50 gelagert wird. Die Lagerelemente 40, 41, 42, 48 und 50 bestehen vorzugsweise jeweils aus Zirkonoxid, vorzugsweise in der mit Yttrium stabilisierten Form, aus Aluminiumoxid, aus einer Keramik oder aus dem gleichen Material wie die Drähte 35, 36 der Antriebswelle 2.

Das Kupplungsgehäuse 4 weist außerdem Kanäle 51 auf zum Zu- und Abführen eines Schmiermediums, wobei die Kanäle mit dem Lagerkanal 49 sowie mit einem Zwischenraum zwischen der Hülse 6 und der Antriebswelle 2 fluidisch leitend verbunden ist. Gemäß dem sechsten Aspekt der Erfindung ist ein Zwischenraum bzw. Zwischenspalt zwischen der Antriebswelle und der Hülse mit einem Schmiermedium, das biokompatibel und vorzugsweise auch physiologisch ist, ausgefüllt. Das Schmiermedium ist biokompatibel und handelt sich in diesem Beispiel um destilliertes Wasser, es könnte aber beispielweise auch eine physiologische Kochsalzlösung oder eine Glucoselösung sein.

Das Kupplungselement 5 der Antriebswelle 2 ist möglichst starr ausgestaltet und dreh-, zug-, und druckfest mit dem proximalen Endstück 3 der Antriebswelle 2 verbunden. Das Kupplungselement 5 der Antriebswelle sowie das Kupplungselement 17 des Antriebsmotors 18, das in diesem Beispiel als eine Aufnahme für das Kupplungselement 5 ausgestaltet ist, weisen zur Ausbildung einer drehfesten aber axial verschiebbaren Verbindung miteinander korrespondierende axiale Gleitflächen 52 bzw. 53 auf. Diese verlaufen parallel zur Längsachse des jeweiligen Kupplungselements 5 bzw. 17 und ändern ihre Form entlang der Längsachse des jeweiligen Kupplungselementes 5 bzw. 17 nicht. In diesem Beispiel handelt es sich bei dem Kupplungselement 5 der Antriebsachse 2 um einen Vierkant.

Der Mantel 7 kann vollständig oder zumindest bereichsweise aus einem Kunststoff bestehen, beispielsweise aus einem Polyurethan, insbesondere aus einem Carbothan oder einem Urethane. Vorzugsweise weist der Mantel eine Metallverstärkung auf, welche beispielsweise aus der für die Antriebswelle vorgeschlagenen Legierung bestehen kann, beispielsweise also aus MP35N^{®}.

Figuren 13 und 14 zeigen jeweils eine schematische perspektivische Darstellung eines Ausführungsbeispiels des in Figur 9 gezeigten ersten Lagerelements 41 des Axiallagers 44. Die Gleitfläche 46 des jeweiligen Lagerelements 41 weist eine Profilierung 55 auf, so dass die beiden Gleitflächen 45, 46 im Zusammenspiel dem Schmiermedium ein hydrodynamisches Gleitlager ausbilden, wodurch ein Abriebsvolumen der Gleitflächen 45, 46 bzw. der beiden Lagerelemente 40, 41 deutlich reduziert werden kann. In den hier gezeigten Ausführungsformen umfasst die Profilierung 55 der jeweiligen Gleitfläche 46 mehrere Erhebungen 56 und Vertiefungen 57. in dem in Figur 13 gezeigten Beispiel sind es genau 12 Erhebungen und 12 Vertiefungen, in dem in Figur 14 gezeigten Beispiel sind es genau 8 Erhebungen und 8 Vertiefungen, wobei die Erhebungen 56 und Vertiefungen 57 jeweils gleichmäßig entlang einer Umlaufrichtung bzw. Umfangrichtung (in den Figuren jeweils mit einem durch U gekennzeichneten Pfeil angedeutet) der jeweiligen Gleitfläche 46 über diese Gleitfläche 46 verteilt angeordnet sind und als eine alternierende Abfolge von Rippen und Rillen ausgestaltet sind.

Diese Rippen und Rillen erstrecken sich jeweils von einem der Antriebswelle 2 zugewandten inneren Rand 58 der jeweiligen Gleitfläche 46 bis zu einem der Antriebswelle 2 abgewandten äußeren Rand 59 der jeweiligen Gleitfläche 46. In dem in Figur 13 gezeigten Beispiel weisen die Rippen jeweils eine Höhe (diese entspricht der Tiefe der jeweils seitlich angrenzenden Rille) von etwa 0,06 mm und eine mittlere Breite (in Umfangrichtung U gemessen) von etwa 0,2 mm. In dem in Figur 13 gezeigten Beispiel weisen die als Rippen ausgestalteten Erhebungen 55 jeweils eine maximale Höhe von etwa 0,1 mm auf, wobei jede Erhebung eine Vorlaufläche 60 und eine Nachlauffläche 61 aufweist, wobei die Vorlaufläche 60 bei einer Drehung des Lagerelements 41 in die bestimmungsgemäße Drehrichtung entlang der Umlaufrichtung U (im Uhrzeigersinn bei einer Blickrichtung zum distalen Ende 9 des Katheters 1) gegenüber der Nachlauffläche 61 voranschreitet.

Diese Vorlauffläche 60 ist gegenüber der Längsachse des Lagerelements 41 derart geneigt bzw. abgeschrägt, so dass sich die Erhebung 56 nach oben hin (d.h. in Richtung der gegenüberliegenden Gleitfläche 45 des zweiten Lagerelements 40, im vorliegenden Beispiel also in die distale Richtung) verschmälert bzw. verjüngt. Mit derartigen geneigten bzw. abgeschrägten Vorlaufflächen 60 kann prinzipiell, also auch in beliebigen anderen Ausführungsbeispielen von Profilierungen des Lagerelements 41, eine gleichmäßigere Bugwellenbildung des Schmiermediums erreicht werden und dadurch ein stabilerer Schmierfilm ausgebildet werden. Auf ihrer jeweiligen Oberseite 62 weist eine jede der Erhebungen 56 eine mittlere Breite (in Umfangrichtung U gemessen) von etwa 0,3 mm auf, wobei sich die Breite der Erhebung 56 in radialer Richtung vergrößert. Eine mittlere Breite (in Umfangrichtung U gemessen) der Rillen 57 beträgt in diesem Beispiel etwa 0,1 mm, wobei sich auch die Breite der Rillen radial nach außen vergrößert. Die in Figuren 13 und 14 gezeigten Ausführungsformen lassen sich beispielsweise mittels eines (Schneid-)Lasers herstellen.

In den Figuren 16 und 17 ist am Beispiels des Werkstoffs 35NLT die Abhängigkeit zwischen den Werkstoffeigenschaften Fließgrenze, Zugfestigkeit, Bruchdehnung und Kaltverfestigungsgrad basierend auf Angaben des Herstellers Fort Wayne Metals dargestellt. Es zeigt sich an diesem Beispiel, dass verschiedene Wärmebehandlungszustände und Kaltverfestigungsgrade eines Werkstoffs generell zu sehr unterschiedlichen Werkstoffeigenschaften führen können.

Bestehen beispielsweise die Antriebswelle 2 und/oder der Hülse 6 des in Figuren 1 bis 15 gezeigten Ausführungsbeispiels aus 35NLT, so liegt der Kaltverfestigungsgrad dieses Werkstoffs vorzugsweise bei etwa 35 bis 70 %, besonders vorzugsweise bei 50 % bis 60 %, damit hier eine Zugfestigkeit von etwa 2000 bis 2200 MPa, beispielsweise 2068 MPa, erzielt und eine Bruchdehnung von 3,5% nicht unterschritten wird.

Die Anmeldung beinhaltet, unter anderem, die folgenden Aspekte:
1. Flexibler Katheter (1) mit einer Antriebswelle (2), einer die Antriebswelle (2) umgebenden Hülse (6) und einem die Antriebswelle (2) und die Hülse (6) umgebender Mantel (7), wobei die Antriebswelle, die Hülse (6) und der Mantel (7) biegsam sind, wobei die Antriebswelle (2) an einem proximalen Ende der Antriebswelle (2) ein Kupplungselement (5) aufweist zum Verbinden der Antriebswelle (2) mit einem Antriebsmotor (18),
   dadurch gekennzeichnet,
   dass die Antriebswelle (2) zumindest bereichsweise aus einer Legierung besteht, die zu jeweils mindestens 10% Gewichtsanteil Chrom, Nickel und Cobalt beinhaltet.
2. Katheter (1) gemäß Aspekt 1, dadurch gekennzeichnet, dass die Legierung zu 30%-40% Gewichtsanteil Nickel, zu 30%-40% Gewichtsanteil Cobalt und/oder zu 15%-25% Gewichtsanteil Chrom beinhaltet.
3. Katheter (1) nach einem der vorangehenden Aspekte, dadurch gekennzeichnet, dass die Legierung zumindest in Bezug auf die Gewichtsanteile von Nickel, Cobalt und Chrom dem Werkstoff MP35N^{®} oder dem Werkstoff 35NLT^{®} entspricht oder um jeweils weniger als 3% Gewichtsanteil hiervon abweicht.
4. Katheter (1) nach einem der vorangehenden Aspekte, dadurch gekennzeichnet, dass die Legierung eine Zugfestigkeit zwischen 1800 N/mm² und 2400 N/mm², vorzugsweise zwischen 2034 N/mm² und 2241 N/mm², aufweist.
5. Katheter (1) nach einem der vorangehenden Aspekte, dadurch gekennzeichnet, dass eine Oberfläche der Antriebswelle (2) eine Rauheit zwischen 0,01 µm und 1µm, vorzugsweise zwischen 0,1 µm und 0,8 µm, aufweist.
6. Katheter (1) gemäß einem der vorangehenden Aspekte, dadurch gekennzeichnet, dass ein Außendurchmesser der Antriebswelle in einem Bereich von etwa 0,53 mm bis etwa 1,32 mm, vorzugsweise in einem Bereich von etwa 0,79 mm bis etwa 0,97 mm, liegt.
7. Katheter (1) nach einem der vorangehenden Aspekte, dadurch gekennzeichnet, dass die Antriebswelle (2) einen sich innerhalb der Antriebswelle (2) axial erstreckenden Hohlraum (29) aufweist.
8. Katheter (1) gemäß Aspekt 7, dadurch gekennzeichnet, dass die Antriebswelle (2) eine Mehrzahl koaxialer Windungen (31, 32) aufweist, die den Hohlraum (29) der Antriebswelle (2) wendelförmig umlaufen.
9. Katheter (1) gemäß Aspekt 8, dadurch gekennzeichnet, dass die Windungen (31, 32) in zwei oder mehr koaxialen Lagen (33, 34) der Antriebswelle (2) angeordnet sind, wobei die Windungen (31, 32) innerhalb verschiedener koaxialer Lagen (33, 34) gegenläufige Wicklungsrichtungen aufweisen.
10. Katheter (1) gemäß einem der Aspekte 8 oder 9, dadurch gekennzeichnet, dass die Windungen (31, 32) durch mindestens einen gewundenen Draht der Antriebswelle (2) gebildet sind, wobei der mindestens eine Draht einen Durchmesser in einem Bereich von etwa 0,09 mm bis etwa 0,21 mm, vorzugweise von etwa 0,135 mm bis etwa 0,165 mm, aufweist.
11. Katheter (1) nach einem der Aspekte 7 bis 10, dadurch gekennzeichnet, dass mindestens ein axialer Abschnitt des Hohlraums (29) der Antriebswelle (2) mit einem Verstärkungsmaterial (30) ausgefüllt ist zum Versteifen der Antriebswelle (2) in dem jeweiligen axialen Abschnitt, vorzugsweise mit einem rostfreien, austenitischen Stahl.
12. Katheter (1) nach einem der vorangehenden Aspekte, dadurch gekennzeichnet, dass ein distales Endstück (19) der Antriebswelle (2) versteift ist, wobei das versteifte distale Endstück (19) vorzugsweise eine Länge zwischen 10 mm bis 60 mm aufweist, besonders bevorzugt eine Länge zwischen 20 mm und 50 mm.
13. Katheter (1) nach einem der vorangehenden Aspekte, dadurch gekennzeichnet, dass das Katheter (1) an einem distalen Ende der Antriebswelle (2) einen fest mit der Antriebswelle (2) verbundenden Pumpenrotor (20) aufweist.
14. Katheter (1) nach Aspekt 13, dadurch gekennzeichnet, dass der Pumpenrotor (20) zur Erzeugung einer nach proximal gerichteten Strömung ausgestaltet ist, wobei der Katheter (1) ein proximal zum Pumpenrotor (20) angeordnetes Axiallager (44) aufweist, das ausgestaltet ist, einer nach distal gerichteten Axialverschiebung der Antriebswelle (2) entgegenzuwirken, oder dass der Pumpenrotor (20) zur Erzeugung einer nach distal gerichteten Axialströmung ausgestaltet ist, wobei der Katheter (1) ein distal zum Pumpenrotor (20) angeordnetes Axiallager (44) aufweist, das ausgestaltet ist, einer nach proximal gerichteten Axialverschiebung der Antriebswelle (2) entgegenzuwirken.
15. Katheter (1) nach Aspekt 14, dadurch gekennzeichnet, dass das Axiallager (44) mindestens ein erstes Lagerelement (41) und ein zweites Lagerelement (40) aufweist, wobei das erste Lagerelement (41) drehfest mit der Antriebswelle (2) verbunden ist und das zweite Lagerelement (40) fest mit der Hülse (6) oder mit dem Mantel (7) verbunden ist, wobei das erste Lagerelement (41) und das zweite Lagerelement (40) einander zugewandte Gleitflächen (45, 46) aufweisen, die eine Axialverschiebung der Antriebswelle (2) bei einem gegenseitigen Berühren in zumindest einer Richtung blockieren.
16. Katheter nach Aspekt 15, dadurch gekennzeichnet, dass die Gleitfläche (46) zumindest des ersten Lagerelements (41) eine Profilierung (55) aufweist zur Ausbildung eines hydrodynamischen Gleitlagers.
17. Katheter nach Aspekt 16, dadurch gekennzeichnet, dass die Profilierung (55) der Gleitfläche (46) mehrere, vorzugsweise 6 bis 24, Erhebungen (56) und/oder Vertiefungen (57) umfasst, die die vorzugsweise jeweils eine Höhe bzw. eine Tiefe von etwa 0,03 mm bis etwa 0,1 mm aufweisen.
18. Katheter nach einem der Aspekte 16 oder 17, dadurch gekennzeichnet, das die Erhebungen (56) und/oder die Vertiefungen (57) als Rippen bzw. als Rillen ausgestaltet sind, die ausgehend von einem der Antriebswelle (2) zugewandten inneren Rand (58) der Gleitfläche (46) sich in Richtung eines der Antriebswelle (2) abgewandten äußeren Randes (59) der Gleitfläche (46) erstrecken, wobei die Rippen bzw. die Rillen vorzugsweise eine Breite in einem Bereich von etwa 0,08 mm bis etwa 0,5 mm aufweisen.
19. Katheter (1) gemäß einem der vorangehenden Aspekte, dadurch gekennzeichnet, dass das Katheter (1) mindestens ein Lagerelement (40, 41, 42, 48, 50) zur radialen und/oder axialen Lagerung der Antriebswelle (2) umfasst, wobei vorzugsweise zumindest eines des mindestens einen Lagerelements (40, 41, 42, 48, 50) zumindest bereichsweise aus Zirkonoxid, insbesondere Yttriumstablisiertem Zirkonoxid, aus Aluminiumoxid, aus einer Keramik und/oder aus der in Aspekt 1, 2 oder 3 genannten Legierung besteht.
20. Katheter (1) gemäß einem der vorangehenden Aspekte, dadurch gekennzeichnet, dass die Hülse (6) als eine Lagerspirale mit einer Vielzahl von Windungen (37) ausgestaltet ist, wobei die Windungen (37) die Antriebswelle (2) in axialer Richtung wendelförmig umlaufen, wobei die Lagerspirale vorzugsweise ein gewickeltes Flachband (38) ist.
21. Katheter (1) gemäß einem der vorangehenden Aspekte, dadurch gekennzeichnet, dass die Hülse (6) zumindest bereichsweise aus demselben Werkstoff wie die Antriebswelle (2) und/oder aus der in Aspekt 1, 2 oder 3 genannten Legierung besteht.
22. Katheter (1) nach einem der vorangehenden Aspekte, dadurch gekennzeichnet, dass ein Zwischenraum zwischen der Antriebswelle (2) und der Hülse (6) mit einem biokompatiblen Schmiermedium ausgefüllt ist, vorzugsweise mit destilliertem Wasser, einer Kochsalzlösung oder einer Glucoselösung.
23. Katheter (1) nach einem der vorangehenden Aspekte, dadurch gekennzeichnet, dass das proximale Kupplungselement (5) der Antriebswelle (2) axiale Gleitflächen (52) aufweist, die entlang einer Längsachse des Kupplungselements (5) gleichförmig ausgestaltet sind für eine drehfeste und axial verschiebbare Verbindung mit dem Antriebsmotor (18).
24. Flexibler Katheter (1) mit einer Antriebswelle (2), einer die Antriebswelle (2) umgebenden Hülse (6) und einem die Antriebswelle (2) und die Hülse (6) umgebender Mantel (7), wobei die Antriebswelle, die Hülse (6) und der Mantel (7) biegsam sind, wobei die Antriebswelle (2) an einem proximalen Ende der Antriebswelle (2) ein Kupplungselement (5) aufweist zum Verbinden der Antriebswelle (2) mit einem Antriebsmotor (18),
   dadurch gekennzeichnet,
   dass die Antriebswelle (2) einen Außendurchmesser von weniger als 1 mm aufweist und zumindest bereichsweise aus einem Material besteht, das eine Zugfestigkeit zwischen zwischen 1800 N/mm² und 2400 N/mm², vorzugsweise zwischen 2034 N/mm² und 2241 N/mm², aufweist.
25. Katheter (1) nach Aspekt 24, dadurch gekennzeichnet, dass eine Oberfläche der Antriebswelle (2) eine Rauheit zwischen 0,01 µm und 1µm, vorzugsweise zwischen 0,1 µm und 0,8 µm, aufweist.
26. Katheter (1) nach einem der Aspekte 24 oder 25, dadurch gekennzeichnet, dass die Antriebswelle (2) und/oder die Hülse (6) zumindest bereichsweise aus einem metallischen Werkstoff bestehen, wobei der metallische Werkstoff vorzugsweise eine Legierung ist, die zu jeweils mindestens 10% Gewichtsanteil Chrom, Nickel und Cobalt beinhaltet.
27. Katheter (1) nach einem der Aspekte 24 bis 26, dadurch gekennzeichnet, dass die Antriebswelle (2) und die Hülse (6) zumindest bereichsweise aus demselben Werkstoff bestehen.
28. Blutpumpenanordnung (16) mit einem Katheter (1) nach einem der vorangehenden Aspekte.
29. Blutpumpenanordnung (16) nach Aspekt 28, wobei die Blutpumpenanordnung (16) außerdem einen Antriebsmotor (18) umfasst, wobei zwischen dem Antriebsmotor (18) und dem Kupplungselement (5) der Antriebswelle (2) eine drehfeste und vorzugsweise axial verschiebbare Verbindung besteht.

### Bezugszeichenliste

- 1: Katheter
- 2: Antriebswelle
- 3: proximales Endstück der Antriebswelle
- 4: Kupplungsmodul
- 5: Kupplungselement der Antriebswelle
- 6: Hülse
- 7: Mantel
- 8: proximales Ende des Katheters
- 9: distales Ende des Katheters
- 10: Pumpenkopf
- 11: Pumpengehäuse
- 12: Abströmschlauch
- 13: Abschlussgehäuse
- 14: Stützelement
- 15: Schleuse
- 16: Blutpumpenanordnung
- 17: Kupplungselement des Antriebsmotors
- 18: Antriebsmotor
- 19: distales Endstück der Antriebswelle
- 20: Pumpenrotor
- 21: Punktionsstelle
- 22: Femoralarterie
- 23: Aortenbogen
- 24: linkes Ventrikel
- 25: Herz
- 26: Innenwand
- 27: Aortenklappe
- 28: Aorta
- 29: Hohlraum
- 30: Verstärkungsmaterial
- 31: Windung der Antriebswelle
- 32: Windung der Antriebswelle
- 33: koaxiale Lage der Antriebswelle
- 34: koaxiale Lage der Antriebswelle
- 35: Draht der Antriebswelle
- 36: Draht der Antriebswelle
- 37: Windung der Hülse
- 38: Flachband
- 39: Innenfläche der Hülse
- 40: Lagerelement
- 41: Lagerelement
- 42: Lagerelement
- 43: Beschaufelung
- 44: Axiallager
- 45: Gleitfläche
- 46: Gleitfläche
- 47: Lagerkanal des Abschlussgehäuses
- 48: Lagerelement
- 49: Lagerkanal des Kupplungsmoduls
- 50: Lagerelement
- 51: Kanal für Schmiermedium
- 52: Gleitfläche
- 53: Gleitfläche
- 54: Kante
- 55: Profilierung
- 56: Erhebung
- 57: Vertiefung
- 58: innerer Rand
- 59: äußerer Rand
- 60: Vorlauffläche
- 61: Nachlauffläche

## Patentansprüche

1. Flexibler Katheter (1), umfassend:
eine Antriebswelle (2), die an einem proximalen Ende der Antriebswelle (2) ein Kupplungselement (5) zum Verbinden der Antriebswelle (2) mit einem Antriebsmotor (18) aufweist und die an einem distalen Ende der Antriebswelle (2) einen fest mit der Antriebswelle (2) verbundenen Pumpenrotor (20) aufweist,
eine die Antriebswelle (2) umgebende Hülse (6),
einen die Antriebswelle (2) und die Hülse (6) umgebenden Mantel (7), und
ein Axiallager (44) zur axialen Lagerung der Antriebswelle (2) und/oder des Pumpenrotors (20),
wobei die Antriebswelle (2), die Hülse (6) und der Mantel (7) biegsam sind.

2. Katheter (1) nach Anspruch 1, **dadurch gekennzeichnet, dass** der Pumpenrotor (20) zur Erzeugung einer nach proximal gerichteten Strömung ausgestaltet ist, wobei das Axiallager (44) proximal zum Pumpenrotor (20) angeordnet und ausgestaltet ist, einer nach distal gerichteten Axialverschiebung der Antriebswelle (2) entgegenzuwirken.

3. Katheter (1) nach Anspruch 1, **dadurch gekennzeichnet, dass** der Pumpenrotor (20) zur Erzeugung einer nach distal gerichteten Axialströmung ausgestaltet ist, wobei das Axiallager (44) distal zum Pumpenrotor (20) angeordnet und ausgestaltet ist, einer nach proximal gerichteten Axialverschiebung der Antriebswelle (2) entgegenzuwirken.

4. Katheter (1) nach einem der vorangehenden Ansprüche, **dadurch gekennzeichnet, dass** das Axiallager (44) mindestens ein erstes Lagerelement (41) und ein zweites Lagerelement (40) aufweist, wobei das erste Lagerelement (41) drehfest mit der Antriebswelle (2) verbunden ist und das zweite Lagerelement (40) fest mit der Hülse (6) oder mit dem Mantel (7) verbunden ist, wobei das erste Lagerelement (41) und das zweite Lagerelement (40) einander zugewandte Gleitflächen (45, 46) aufweisen, die eine Axialverschiebung der Antriebswelle (2) bei einem gegenseitigen Berühren in zumindest einer Richtung blockieren.

5. Katheter (1) nach Anspruch 4, **dadurch gekennzeichnet, dass** die Gleitfläche (46) zumindest des ersten Lagerelements (41) eine Profilierung (55) aufweist zur Erzeugung von Druck- oder Bugwellen eines Schmiermediums zwischen den Gleitflächen (45, 46) des ersten und zweiten Lagerelements (40, 41) im Drehbetrieb der Antriebswelle (2).

6. Katheter (1) nach Anspruch 5, **dadurch gekennzeichnet, dass** die Profilierung (55) mehrere Erhebungen (56) und/oder Vertiefungen (57) umfasst.

7. Katheter (1) nach Anspruch 6, **dadurch gekennzeichnet, dass** die Erhebungen (56) und/oder Vertiefungen (57) jeweils eine Höhe bzw. eine Tiefe von etwa 0,03 mm bis etwa 0,1 mm aufweisen.

8. Katheter (1) nach einem der Ansprüche 6 oder 7, **dadurch gekennzeichnet, dass** die Erhebungen (56) und/oder die Vertiefungen (57) als Rippen bzw. als Rillen ausgestaltet sind, die ausgehend von einem der Antriebswelle (2) zugewandten inneren Rand (58) der Gleitfläche (46) sich in Richtung eines der Antriebswelle (2) abgewandten äußeren Randes (59) der Gleitfläche (46) erstrecken

9. Katheter (1) nach Anspruch 9, **dadurch gekennzeichnet, dass** die Rippen und/oder die Rillen eine Breite in einem Bereich von etwa 0,08 mm bis etwa 0,5 mm aufweisen.

10. Katheter (1) nach einem der Ansprüche 6 bis 10, **dadurch gekennzeichnet, dass** die Erhebungen (54) Vorlaufflächen (60) aufweisen, die gegenüber einer Längsachse des ersten Lagerelements (41) derart geneigt sind, dass sich die Erhebungen (54) in Richtung zur gegenüberliegenden Gleitfläche (45) des zweiten Lagerelements (40) hin verjüngen.

11. Katheter (1) gemäß einem der vorangehenden Ansprüche, **dadurch gekennzeichnet, dass** das erste Lagerelement (41) und/oder das zweite Lagerelement (40) zumindest bereichsweise aus Zirkonoxid, insbesondere Yttriumstablisiertem Zirkonoxid, Aluminiumoxid, einer Keramik und/oder aus einer Legierung besteht, wobei die Legierung zu jeweils mindestens 10% Gewichtsanteil Chrom, Nickel und Cobalt beinhaltet.

12. Katheter (1) nach einem der vorangehenden Ansprüche, **dadurch gekennzeichnet, dass** ein Zwischenraum zwischen der Antriebswelle (2) und der Hülse (6) mit einem biokompatiblen Schmiermedium ausgefüllt ist.

13. Katheter (1) nach Anspruch 12, **dadurch gekennzeichnet, dass** Schmiermedium destilliertes Wasser, eine Kochsalzlösung oder eine Glucoselösung ist.

14. Katheter (1) nach einem der vorangehenden Ansprüche, **dadurch gekennzeichnet, dass** das proximale Kupplungselement (5) der Antriebswelle (2) axiale Gleitflächen (52) aufweist, die entlang einer Längsachse des Kupplungselements (5) gleichförmig ausgestaltet sind für eine drehfeste und axial verschiebbare Verbindung mit dem Antriebsmotor (18).

15. Blutpumpenanordnung (16), umfassend:
einen Katheter (1) nach einem der vorangehenden Ansprüche und
einen Antriebsmotor (18), wobei zwischen dem Antriebsmotor (18) und dem Kupplungselement (5) der Antriebswelle (2) eine drehfeste und vorzugsweise axial verschiebbare Verbindung herstellbar ist.
